(19) Europäisches Patentamt
European Patent Office
Office européen des brevets

(11) Numéro de publication: **0 234 980 B1**

(12) # FASCICULE DE BREVET EUROPEEN

(45) Date de publication de fascicule du brevet: **16.09.92**

(51) Int. Cl.[5]: **C07D 403/12**, C07D 401/12, A61K 31/415, A61K 31/44, A61K 31/505

(21) Numéro de dépôt: **87400112.6**

(22) Date de dépôt: **19.01.87**

Le dossier contient des informations techniques présentées postérieurement au dépôt de la demande et ne figurant pas dans le présent fascicule.

(54) **Dérivés de 2-benzimidazolylalkylthio (ou sulfinyle ou sulfonyle) leur préparation et leur application en tant que médicaments.**

(30) Priorité: **20.01.86 FR 8600695**

(43) Date de publication de la demande: **02.09.87 Bulletin 87/36**

(45) Mention de la délivrance du brevet: **16.09.92 Bulletin 92/38**

(84) Etats contractants désignés: **AT BE CH DE FR GB GR IT LI LU NL SE**

(56) Documents cités:
**FR-A- 2 261 007**

**EUROPEAN JOURNAL OF MEDICINAL CHEMISTRY, vol. XVIII, no. 3, 1983, pages 277-285, Châtenay-Malabry, FR; D.E. BEATTIE et al.: "Anti-ulcer and gastric antisecretory activity of a series of thioethers and related sulphoxides"**

(73) Titulaire: **LABORATORIOS DEL DR. ESTEVE, S.A.**
**Av. Mare de Deu de Montserrat, 221**
**E-08026 Barcelona(ES)**

(72) Inventeur: **Constansa, Jordi Frigola**
**Av. Diagonal, 299 at. 1a**
**E-08013 Barcelone(ES)**
Inventeur: **Pinol, Augusto Colombo**
**Av. Chile, 36, 4o 1a**
**E-08032 Barcelone(ES)**
Inventeur: **Corominas, Juan Parés**
**Padilla, 349, 3o 3a**
**E-08025 Barcelone(ES)**

(74) Mandataire: **Ahner, Francis et al**
**CABINET REGIMBEAU, 26, avenue Kléber**
**F-75116 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à de nouveaux dérivés de benzimidazole, leur procédé de préparation, ainsi que leur application en tant que médicaments.

Les composés objet de la présente invention peuvent également être utilisés dans l'industrie pharmaceutique comme intermédiaires et pour la préparation de médicaments.

Les effets inhibiteurs de la sécrétion d'acide gastrique sont bien documentés (voir en particulier DE-A-3 404 610 ; US-A-4 472 409 ; EP-A-0 074341 ; EP-A-0 045 200 ; EP-A-0 005 129 pour les dérivés de 2-benzimidazolylthio (ou sulfinyle) alkyl-pyridines de formule générale

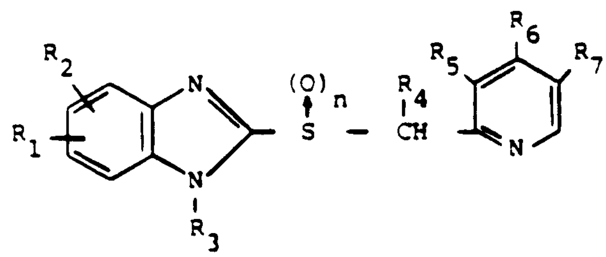

dans laquelle n représente 0 ou 1.

Les nouveaux dérivés de 2-benzimidazolylalkylthio (ou sulfinyle ou sulfonyle)pyridines (ou pyrimidines), objet de la présente invention, répondent à la formule générale I :

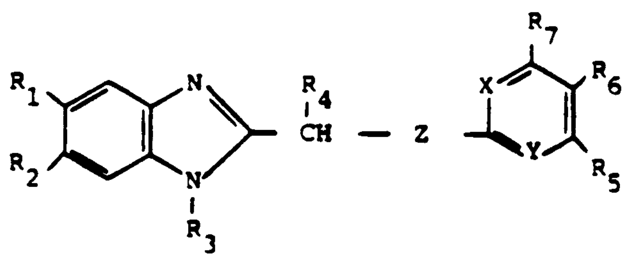

(I)

dans laquelle :

| | |
|---|---|
| X | représente un atome d'azote (N) ou un atome de carbone lié à un autre radical $R_8$ ($C-R_8$); |
| Y | représente un atome d'azote (N) ou un groupe N - oxyde ($N \rightarrow O$); |
| Z | représente un atome de soufre (S), un groupe sulfinyle ($S \rightarrow O$) ou un groupe sulfonyle ($O \leftarrow S \rightarrow O$); |
| $R_1$ et $R_2$ | identiques ou différents, représentent un atome d'hydrogène, un halogène, un radical alkyle inférieur linéaire ou ramifié en $C_1$ à $C_4$, un groupe nitro ($NO_2$), un groupe trifluorométhyle ($CF_3$), un radical alkoxy inférieur en $C_1$ à $C_4$, un radical méthyl carbonyle, méthoxycarbonyle, benzoyle ; |
| $R_3$ | représente un atome d'hydrogène, un radical alkyle inférieur en $C_1$ à $C_4$, un radical carbonyle lié à un autre radical $R_9$ 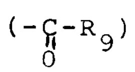 ; |
| $R_4$ | représente un atome d'hydrogène, un radical alkyle inférieur en $C_1$ à $C_4$ ; |
| $R_5$ | représente un atome d'hydrogène, un radical méthyle, un radical hydroxy; |
| $R_6$ | représente un atome d'hydrogène, un radical méthyle, un radical nitro ($NO_2$); |
| $R_7$ | représente un atome d'hydrogène, un radical alkyle inférieur en $C_1$ à $C_4$; |
| $R_8$ | représente un atome d'hydrogène, un radical méthyle; |
| $R_9$ | représente un radical alkoxy; |

à l'exception toutefois du composé de formule I, dans laquelle:

X représente CH,

Y représente N,
Z représente S, et
$R_1$ à $R_7$ représentent H.

La presente invention se rapporte également aux sels physiologiquement acceptables des composés de formule générale I.

Les dérivés de formule générale I et leurs sels sont apropriés pour prévenir ou traiter les maladies gastro-intestinales chez les mammifères, dont l'homme, principalement la sécrétion d'acide gastrique et la capacité cytoprotectrice.

La 2-(2-benzimidazolymethylthio)pyridine a été décrite, notamment dans FR-A-2 261 007, sans que ses propriétés de traitement des maladies gastro-intestinales ne soient toutefois quantifiées.

Les dérivés de formule générale I peuvent également être utilisés dans l'industrie pharmaceutique comme intermédiaires et pour la préparation de médicaments.

Les nouveaux dérivés de formule générale I peuvent être préparés conformément à l'invention, selon les méthodes suivantes :

Méthode A

Par réaction d'un composé de formule générale II :

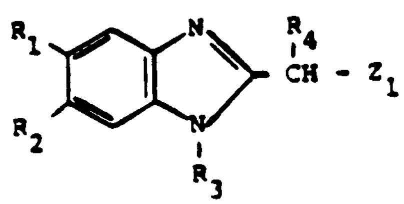

(II)

avec un composé de formule générale III

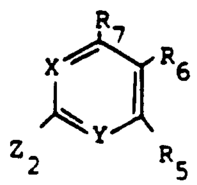

(III)

formules dans lesquelles X,Y, et $R_1$ à $R_7$ ont les significations mentionnées précédemment, et l'un des deux radicaux $Z_1$ et $Z_2$ est constitué par le radical -SH et l'autre est un groupe partant notamment choisi parmi les halogènes, de préférence le fluor, le chlore ou le brome ; des radicaux formés par des groupes estérifiés et qui soient réactifs notamment acétyloxy, tosyloxy ou mésyloxy ; ou encore des groupes alkylthio ou alkylsulfinyle, par exemple méthylthio ou méthylsulfinyle.

La réaction d'un composé de formule II avec un composé de formule III s'effectue en présence d'un solvant adéquat, par exemple, des alcools tels que le méthanol ou l'éthanol ; des mélanges de ces alcools avec de l'eau ; des éthers comme par exemple le tétrahydrofuranne. Cette réaction est avantageusement conduite en présence d'une base adéquate, tant des bases minérales comme l'hydroxide de sodium ou l'hydrure de sodium, que des bases organiques telles que les amines tertiaires. La réaction entre ces deux composés s'effectue entre environ -5 ° C et la température d'ébullition du mélange réactionnel.

Dans les composés préparés, de formule générale I, X, Y et $R_1$ à $R_7$ ont les significations mentionnées précédemment et Z représente un atome de soufre.

Méthode B

Par réaction d'un composé de formule générale II avec un composé de formule générale III, dans lesquelles X et $R_1$ à $R_7$ ont les significations mentionnées précédemment, Y représente un groupe N-oxyde (N → O), $Z_1$ représente -SH et $Z_2$ représente un atome d'hydrogène.

La réaction entre ces deux composés s'effectue de préférence dans de l'anhydride acétique à des températures comprises entre 60 ° C et la température d'ébullition du mélange réactionnel. Dans les

composés préparés, de formule générale I, X et $R_1$ à $R_7$ ont les significations mentionnées précédemment, Y représente un atome d'azote et Z représente un atome de soufre.

Méthode C

Par réaction d'un composé de formule générale IV :

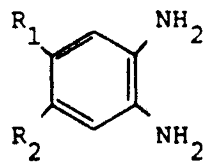

(IV)

avec un composé de formule générale V :

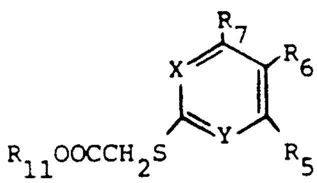

(V)

dans lesquelles : X, Y, $R_1$, $R_2$, $R_5$, $R_6$ et $R_7$ ont les significations mentionnées précédemment et $R_{11}$ représente un atome d'hydrogène ou un radical alkyle inférieur tel que méthyle ou éthyle.

La réaction d'un composé de formule générale IV avec un composé de formule générale V s'effectue de préférence dans un acide minéral, tel que l'acide chlorhydrique ou l'acide polyphosphorique, à des températures entre environ 60 ° C et environ 150 ° C sous atmosphère d'azote.

Dans les composés préparés, de formule générale I, X, Y, $R_1$, $R_2$, $R_5$, $R_6$ et $R_7$ ont les significations mentionnées précédemment, $R_3$ et $R_4$ représentent un atome d'hydrogène et Z représente un atome de soufre.

Méthode D

Par oxydation, avec une quantité stoechiométrique de l'agent oxydant, des composés de formule générale I, dans laquelle X, Y et $R_1$ à $R_7$ ont les significations mentionnées précédemment et Z représente un atome de soufre, on prépare des composés de formule générale I dans laquelle X, Y et $R_1$ à $R_7$ ont les significations mentionnées précédemment et Z représente un groupe sulfinyle ($S \rightarrow O$).

L'oxydation de l'atome de soufre en groupe sulfinyle s'effectue de préférence en présence d'un agent oxydant sélectionné parmi : peroxyde d'hydrogène, peracides tels que m-chloroperbenzoïque, acide nitrique, métapériodate de sodium, acide chromique, dioxyde de manganèse, chlore, brome ou chlorure de sulfuryle. Les différents solvants utilisés peuvent être acide acétique, alcools tels que méthanol ou éthanol, et solvants chlorés tels que chlorure de méthylène ou chloroforme. L'oxydation s'effectue à des températures entre environ -70 ° C et environ 50 ° C, de préférence entre environ -20 ° C et environ 30 ° C.

Certains des nouveaux composés peuvent être présents comme isomères optiques ou racémates, dépendant du produit de départ et du procédé. Dans le cas où $R_4$ représente un substituant différent de l'hydrogène, il y a deux centres chiraux

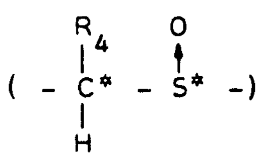

qui donnent lieu par conséquent, à un mélange racémique qui peut être séparé en deux racémates purs diastéréoisomériques au moyen de procédés chromatographiques ou par cristallisation fractionnée. Les

racémates obtenus peuvent se séparer pour obtenir leurs énantiomères en utilisant des acides optiquement actifs de façon à former des sels qui peuvent se séparer par différence de solubilités, recristallisation avec un solvant optiquement actif ou au moyen de microorganismes.

Méthode E

Par oxydation des composés de formule générale I dans laquelle X, Y et $R_1$ à $R_7$ ont les significations mentionnées précédemment et Z représente un atome de soufre, avec une quantité deux à trois fois supérieure de l'agent oxydant, on prépare des composés de formule générale I dans laquelle X, Y et $R_1$ à $R_7$ ont les significations mentionnées précédemment et Z représente un groupe sulfonyle (O ← S → O).

Les agents oxydants et les solvants sont les mêmes que ceux décrits dans la méthode D. La réaction d'oxydation s'effectue à température ambiante ou à des températures supérieures, jusqu'à atteindre le point d'ébullition du solvant.

Méthode F

Par nitration, dans un mélange d'acides sulfurique et nitrique, des composés de formule générale I dans laquelle $R_2$ et $R_3$ représentent des atomes d'hydrogène et X, Y, Z, $R_1$, $R_4$ à $R_7$ ont les significations mentionnées précédemment, on prépare des composés de formule générale I dans laquelle X, Y, Z, $R_1$ et $R_4$ à $R_7$ ont les significations mentionnées précédemment, $R_2$ représente un groupe nitro ($NO_2$) et $R_3$ représente un atome d'hydrogène.

La réaction de nitration s'effectue à des températures comprises entre -5 ° C et 30 ° C, pendant un temps compris entre 1 heure et 6 heures.

Méthode G

Pour la préparation des composés de formule générale I où $R_1$ ou $R_2$ est méthoxycarbonyle ($COOCH_3$) , $R_3$ représente un atome d'hydrogène et X, Y, Z, $R_1$ ou $R_2$ et $R_4$ à $R_7$ ont les significations précédemment mentionnées, on fait réagir un composé de formule générale I dans laquelle $R_1$ ou $R_2$ représente un radical carboxylique (COOH), $R_3$ représente un atome d'hydrogène et $R_1$ ou $R_2$, X, Y, Z et $R_4$ à $R_7$ ont les significations précédemment mentionnées, avec du méthanol.

La réaction d'estérification s'effectue en utilisant comme solvant l'alcool et en présence d'un acide minéral tel que l'acide chlorhydrique ou d'un acide organique tel que l'acide p-toluènesulfonique, à température ambiante ou à des températures supérieures jusqu'à atteindre le point d'ébullition de l'alcool, pendant un temps compris entre 2 jours et deux heures.

Méthode H

Pour la préparation des composés de formule générale I dans laquelle $R_3$ représente un radical alkyl inférieur en $C_1$ à $C_4$ et X, Y, Z, $R_1$, $R_2$ et $R_4$ à $R_7$ ont les significations précédemment mentionnées, on fait réagir un composé de formule générale I dans laquelle $R_3$ représente un atome d'hydrogène et X, Y, Z, $R_1$, $R_2$ et $R_4$ à $R_7$ ont les significations précédemment mentionnées, avec un agent alkylant tel que sulfate de diméthyle, diméthylformamide diméthyl acétal ou un halogénure d'alkyle dans lequel les halogènes préférés sont le chlore, le brome et l'iode.

La réaction d'alkylation s'effectue au sein d'un solvant adéquat, par exemple, alcools tels que méthanol ou éthanol ; cétones telles que diméthylcétone ; mélange de ces alcools ou cétones avec de l'eau ; solvants inertes tels que toluène ; solvants polaires aprotiques tels que diméthylformamide. Les meilleures conditions pour effectuer cette réaction, c'est d'opérer en présence d'une base appropriée (excepté lorsqu'on utilise comme réactif le diméthylformamide diméthylacétal) tant des bases minérales tel qu'hydroxyde de potassium, carbonate de sodium ou carbonate de potassium, que des bases organiques tel que des amines tertiaires.

La réaction d'alkylation s'effectue à température ambiante ou à des températures plus élevées, jusqu'à atteindre la température d'ébullition du solvant, dans un temps compris entre 2 et 24 heures.

On peut également effectuer l'alkylation dans des conditions de transfert de phase en utilisant comme catalyseur un sel d'ammonium quaternaire, de préférence le sulfate acide de tétrabutylammonium.

Dans les composés où $R_1$ est différent de $R_2$, on obtient, dans certains cas, deux isomères de position qui peuvent être séparés par cristallisation fractionnée ou par des méthodes chromatographiques.

Méthode I

Pour la préparation des composés de formule générale I dans laquelle $R_3$ représente un radical carbonyle lié à un autre radical $R_9$

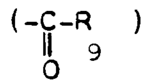

et X, Y, Z, $R_1$, $R_2$ et $R_4$ à $R_9$ ont les significations précédemment mentionnées, on fait réagir un composé de formule générale I dans laquelle $R_3$ représente un atome d'hydrogène et X, Y, Z, $R_1$, $R_2$ et $R_4$ à $R_7$ ont les significations précédemment mentionnées, avec un composé de formule VI :

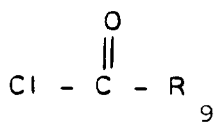

VI

dans laquelle $R_9$ a la signification précédemment mentionnée.

La réaction s'effectue au sein d'un solvant approprié, par exemple cétones tels que diméthylcétone ; éthers tels que tétrahydrofuranne ; mélanges de cétones avec l'eau ; solvants chlorés tels que chlorure de méthylène ; solvants polaires aprotiques tels que diméthylformamide ou diméthylsulfoxyde. Les meilleures conditions pour réaliser cette réaction, c'est d'opérer en présence d'une base appropriée, tant une base minérale telle qu'hydrure de sodium, carbonate de sodium ou carbonate de potassium, que base organique telle que triéthylamine. Les températures les plus adéquates oscillent entre environ -5 ° C et environ 35 ° C, le temps de réaction étant compris entre 1 heure et 24 heures.

Pour les composés où $R_1$ est différent de $R_2$, on obtient, dans certains cas, deux isomères de position qui peuvent être séparés par cristallisation fractionnée ou par des méthodes chromatographiques.

Les composés de formule générale I, conformément à l'invention, peuvent être synthétisés tant sous forme de base libre que d'un sel, suivant les conditions de la réaction et la nature des produits de départ. Les sels peuvent se transformer en base libre en utilisant des agents basiques tels qu'alcalis et carbonates alcalins ou par échange ionique. D'autre part, les bases libres synthétisées peuvent à leur tour former des sels avec des acides minéraux ou organiques.

Les composés de formule générale I, conformément à l'invention sont synthétisés de préférence selon la méthode A, et ses sulfures optionnellement oxydés selon les méthodes D et E ; l'alkylation ou l'acylation suivante peut être effectuée respectivement selon les méthodes H ou I.

Les composés de formules générales II, III, IV et V sont connus ou peuvent être préparés par des procédés analogues à des procédés connus à partir de composés facilement accessibles. Ainsi, par exemple, les composés de formule générale II se synthétisent par réaction des composés de formule générale IV avec des acides alpha-mercaptoalcanoïques [E.S.Milner, jun., S. Snyder, M.M. Joullié, J.Chem.Soc. 1964, 4151 ; P.Lochou, J.Schoenleber, Tetrahedron, 1976, 32, 2023] ou bien avec des acides alpha-hydroxyalcanoïques et, dans ce cas, traitement optionnel suivant avec du chlorure de tosyle, chlorure de mésyle ou chlorure de thionyle [W.R. Siegart, A.R. Day, J.Amer. Chem.Soc., 1957, 79, 4391]. Les composés de formule générale V se synthétisent par réaction des composés de formule générale III avec l'acide thioglycolique ou un thioglycolate d'alkyle lorsque $Z_2$ représente un des "leaving groups" indiqués dans la méthode A ou de l'hydrogène, et dans ce dernier cas Y représente N → O [F.M. Hershenson, L.Bauer, J.Org.Chem., 1969, 34, 655]. On peut aussi synthétiser les composés de formule générale V par réaction des composés de formule générale III lorsque $Z_2$ représente SH, avec un chloroacétate d'alkyle ou un bromoacétate d'alkyle, dans un milieu basique.

Dans les exemples suivants on indiquera la préparation de nouveaux dérivés selon l'invention. On décrira également quelques formes d'emploi typiques pour les différents domaines d'application.

EXEMPLE 1

Méthode A : Préparation de 2-(2-benzimidazolylméthylthio)-pyrimidine

On ajoute 10,0 g (0,06 mole) de 2-chlorométhylbenzimidazole à une solution de 6,7 g (0,06 mole) de 2-mercaptopyrimidine et 2,4 g (0,06 mole) d'hydroxyde de sodium dans 200 ml d'éthanol et 20 ml d'eau. On maintient à reflux pendant 2 heures et on évapore l'éthanol. On reprend le résidu avec de l'eau (50 ml) et on extrait avec de l'acétate d'éthyle (3 x 50 ml), on sèche la phase organique avec du sulfate de sodium, on filtre et on évapore jusqu'à un volume de 40 ml de solution dans laquelle cristallisent 10,2 g (70%) de 2-(2-benzimidazolylméthylthio)-pyrimidine, de point de fusion 154-155 °C.

Les données pour l'identification du produit se présentent dans les tableaux 1 et 2.

EXEMPLES 2 à 18 et 21 à 26

Les composés identifiés par les exemples 2 à 18 et 21 à 26 dans les tableaux 1 et 2, sont généralement préparés par les méthodes A ou C. Les composés des exemples 6, 10 et 21 à 26 peuvent aussi être préparés par la méthode B.

EXEMPLE 6

Méthode B : Préparation de 5,6-diméthyl-2-(2-pyridyl-thiométhyl)-benzimidazole

On chauffe à 100 °C pendant 4 heures une solution de 3,84g (0,02 mole) de 5,6-diméthyl-2-mercaptométhyl-benzimidazole et 1,9 g (0,02 mole) de pyridine N-oxyde dans 50 ml d'anhydride acétique. On évapore l'anhydride acétique et on reprend avec de l'acétone, on filtre et on concentre jusqu'à un volume de 20 ml. De cette solution cristallisent 2,6 g (48%) de 5,6-diméthyl-2-(2-pyridylthiométhyl)-benzimidazole. Le produit ainsi obtenu est identique à celui obtenu par la méthode A, et les données pour l'identification se présentent dans les tableaux 1 et 2.

EXEMPLE 10

Méthode C : Préparation de 5-benzoyl-2-(2-pyridylthiométhyl)-benzimidazole

On chauffe à reflux pendant 2 jours 8,45 g d'acide 2-(2-pyridylthio)-acétique (0,05 mole) et 10,6g de 4-benzoyl-1,2-phénylènediamine (0,05 mole) dans 100 ml d'acide chlorhydrique 4N. On laisse refroidir le mélange et on neutralise avec de l'ammoniaque. On extrait avec de l'acétate d'éthyle (3 x 100 ml), on sèche ($Na_2SO_4$), on filtre et on concentre jusqu'à un volume de 125 ml. De cette solution cristallisent 11,7 g (68%) de 5-benzoyl-2-(2-pyridylthiométhyl)-benzimidazole. Le produit ainsi obtenu est identique à celui obtenu par la méthode A, et les données pour l'identification se présentent dans les tableaux 1 et 2.

EXEMPLE 22

Méthode C : Préparation de 5-methoxy-2(4-méthyl-2-pyridylthiométhyl)-benzimidazole

On chauffe à reflux pendant 2 jours 10,6 g de 2-(4-méthyl-2-pyridylthio)-acétate d'éthyle (0,05 mole) et 8,8 g de 4-méthoxy-1,2-phénylènediamine chlorhydrate (0,05 mole) dans 400 ml d'acide chlorhydrique 6N. On laisse refroidir le mélange et on neutralise avec de l'ammoniaque. On extrait avec de l'acétate d'éthyle (3 X 100 ml), on sèche ($Na_2SO_4$),on filtre et on concentre jusqu'à un volume de 75 ml. De cette solution cristallisent 8,4 g (59 %) de 5-méthoxy-2-(4-méthyl-2-pyridylthiométhyl)-benzimidazole, de point de fusion 138-140 °C. Les données pour l'identification du produit se présentent dans les tableaux 1 et 2.

TABLEAU 1

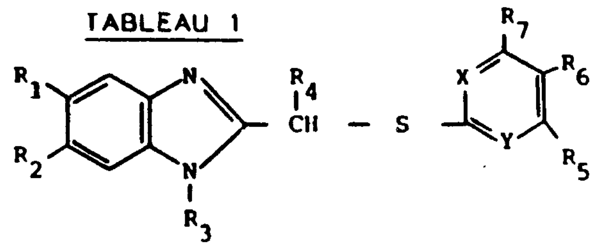

| Exemple n° | X | Y | $R_1$ | $R_2$ | $R_3$ | $R_4$ | $R_5$ | $R_6$ | $R_7$ | p.f. °C | IR (KBr) $cm^{-1}$ |
|---|---|---|---|---|---|---|---|---|---|---|---|
| 1 | N | N | H | H | H | H | H | H | H | 154-5 | 750 (Base) |
| 2 | N | N | H | H | H | H | OH | H | $CH_2CH_2CH_3$ | 234-6 | 740 (Base) |
| 3 | N | N | H | H | H | H | $CH_3$ | H | $CH_3$ | 164-7 | 750 (Base) |
| 4 | CH | N | H | H | H | H | H | $NO_2$ | H | 185-7 | 750;1340; 1510 (Base) |
| 5 | CH | N | $CH_3$ | H | H | H | H | H | H | 133-5 | 755 (Base) |
| 6 | CH | N | $CH_3$ | $CH_3$ | H | H | H | H | H | 148-51 | 750 (Base) |
| 7 | CH | N—O | H | H | H | H | H | H | H | 193-6 | 730 (Base) |
| 8 | CH | N | Cl | H | H | H | H | H | H | 131-4 | 750 (Base) |
| 9 | CH | N | $CF_3$ | H | H | H | H | H | H | 121-7 | 765 (HCl) |
| 10 | CH | N | $C_6H_5CO-$ | H | H | H | H | H | H | 129-34 | 760;1665 (HCl) |
| 11 | CH | N | $CH_3O-$ | H | H | H | H | H | H | 136-45 | 770 (Base) |
| 12 | CH | N | H | H | H | $CH_3$ | H | H | H | 173-5 | 750 (Base) |
| 13 | CH | N | H | H | H | H | H | H | H | 158 | 750 (Base) |

TABLEAU 1 (Continuation)

| Exemple n° | X | Y | $R_1$ | $R_2$ | $R_3$ | $R_4$ | $R_5$ | $R_6$ | $R_7$ | p.f. °C | IR (KBr) $cm^{-1}$ |
|---|---|---|---|---|---|---|---|---|---|---|---|
| 14 | $C-CH_3$ | N | $C_6H_5CO-$ | H | H | H | H | $CH_3$ | H | 69-73 | 730;1660 (Base) |
| 15 | $C-CH_3$ | N | $C_6H_5CO-$ | H | H | H | H | H | H | 103-8 | 750;1660 (Base) |
| 16 | $C-CH_3$ | N | H | H | H | H | H | H | H | 133-7 | 740 (Base) |
| 17 | CH | N | H | H | H | H | H | H | $CH_3$ | 147-50 | 740 (Base) |
| 18 | CH | N | $CF_3$ | H | H | H | H | H | $CH_3$ | 87-92 | 810 (Base) |
| 19 | CH | N | $CH_3OOC-$ | H | H | H | H | H | $CH_3$ | 121-4 | 750;1725 (HCl) |
| 20 | CH | N | $CH_3OOC-$ | H | H | H | H | H | H | 160-2 | 750;1710 (HCl) |
| 21 | CH | N | $C_6H_5CO-$ | H | H | H | H | H | $CH_3$ | 165-8 | 825;1665 (HCl) |
| 22 | CH | N | $CH_3O$ | H | H | H | H | H | $CH_3$ | 138-40 | 800 (Base) |
| 23 | CH | N | $CH_3CO-$ | H | H | H | H | H | $CH_3$ | 141-4 | 1695;840 (HCl) |
| 24 | CH | N | $CH_3$ | H | H | H | H | H | $CH_3$ | 139-41 | 810 (Base) |
| 25 | CH | N | $CH_3$ | $CH_3$ | H | H | H | H | $CH_3$ | 156-9 | 805 (Base) |
| 26 | CH | N | Cl | H | H | H | H | H | $CH_3$ | 147-9 | 795 (Base) |

## TABLEAU 2

| Exemple n° | $^1H$ RMN $\delta$ [ DMSO - $d_6$ ] |
|---|---|
| 1 | 8,64 (d,2H); 7,58-7,10 (m,6H); 4,72 (s,2H) |
| 2 | 7,56-7,05 (m,4H); 7,20 (é,2H); 6,01 (s,1H); 4,64 (s,2H); 2,43 (t,2H); 1,58 (Sext.,2H); 0,84 (t,3H) |
| 3 | 7,51 (m,2H); 7,14 (m,2H); 6,89 (s,1H); 6,52 (é,1H); 4,67 (s,2H); 2,34 (s,6H) |
| 4 | 9,17 (s,1H); 8,30 (m,1H); 7,65-7,00 (m,6H); 4,8[illegible] (s,2H) |
| 5 | 10,53 (é,1H); 8,47 (d,1H); 7,70-6,94 (m,6H); 4,70 (s,2H); 2,39 (s,3H) |
| 6 | 8,46 (d,1H); 7,75-7,05 (m,5H); 6,05 (é,1H); 4,62 (s,2H); 2,27 (s,6H) |
| 7 | 8,45-6,90 (m,9H); 4,56 (s,2H) |
| 8 | 8,47 (d,1H); 7,70-7,00 (m,7H); 4,69 (s,2H) |
| 9 | 12,82 (é,1H); 8,47 (d,1H); 7,90-7,00 (m,6H); 4,73 (s,2H) |
| 10 | 12,75 (é,1H); 8,46 (d,1H); 7,90-7,00 (m,11H); 4,73 (s,2H) |
| 11 | 13,41 (é,1H); 8,48 (d,1H); 7,77-6,74 (m,6H); 4,64 (s,2H); 3,76 (s,3H) |
| 12 | 12,31 (é,1H); 8,50 (d,1H); 7,72-7,00 (m,7H); 5,42 (q,1H); 1,87 (d,3H) |
| 13 | 12,00 (é,1H); 8,48 (d,1H); 7,70-7,00 (m,7H); 4,73 (s,2H) |

**TABLEAU 2 (Continuation)**

| Exemple n° | $^{1}H$ RMN $\delta$ [DMSO - $d_6$] |
|---|---|
| 14 | 9,00 (é,1H); 8,17 (s,1H); 7,87 (s,1H); 7,62 (m,7H); 7,33 (s,1H); 4,71 (s,2H); 2,28 (s,3H); 2,22 (s,3H) |
| 15 | 8,30 (d,1H); 8,00 (s,1H); 7,84-7,53 (m,9H); 7,12 (m,1H); 4,92 (s,2H); 2,26 (s,3H) |
| 16 | 8,37 (d,1H); 7,61 (é,1H); 7,52 (m,3H); 7,15 (m,3H); 4,74 (s,2H); 2,22 (s,3H) |
| 17 | 10,15 (é,1H); 8,34 (d,1H); 7,53 (m,2H); 7,21 (m,3H); 6,94 (d,1H); 4,71 (s,2H); 2,24 (s,3H) |
| 18 | 12,71 (é,1H); 8,33 (d,1H); 7,86 (s,1H); 7,69 (d,1H); 7,45 (d,1H); 7,22 (s,1H); 6,95 (d,1H); 4,71 (s,2H); 2,24 (s,3H) |
| 19 | 9,52 (é,1H); 8,32 (d,1H); 8,17 (s,1H); 7,82 (d,1H); 7,59 (d,1H); 7,19 (s,1H); 6,91 (d,1H); 4,72 (s,2H); 3,85 (s,3H); 2,21 (s,3H) |
| 20 | 12,62 (é,1H); 8,49 (d,1H); 8,14 (s,1H); 7,92-7,37 (m,4H); 7,15 (t,1H); 4,71 (s,2H); 3,87 (s,3H) |
| 21 | 10,40 (é,1H); 8,32 (d,1H); 7,97 (s,1H); 7,61 (m,7H); 7,18 (s,1H); 6,90 (d,1H); 4,75 (s,2H); 2,20 (s,3H) |
| 22 | 12,09 (é,1H); 8,33 (d,1H); 7,37 (d,1H); 7,23 (s,1H); 7,00 (m,2H); 6,75 (d,1H); 4,58 (s,2H); 3,76 (s,3H); 2,26 (s,3H) |
| 23 | 12,50 (é,1H); 8,31 (d,1H); 8,14 (s,1H); 7,79 (d,1H); 7,55 (d,1H); 7,19 (s,1H); 6,93 (d,1H); 4,68 (s,2H); 2,58 (s,3H); 2,23 (s,3H) |
| 24 | 11,74 (é,1H); 8,35 (d,1H); 7,39 (m,2H); 7,22 (s,1H); 6,97 (m,2H); 4,70 (s,2H); 2,41 (s,3H); 2,24 (s,3H) |
| 25 | 10,13 (é,1H); 8,33 (d,1H); 7,29 (s,2H); 7,20 (s,1H); 6,92 (s,1H); 4,66 (s,2H); 2,30 (s,6H); 2,24 (s,3H) |
| 26 | 10,70 (é,1H); 8,32 (d,1H); 7,56 (m,2H); 7,19 (m,2H); 6,92 (d,1H); 4,70 (s,2H); 2,23 (s,3H) |

EXEMPLE 27

Méthode D : Préparation de 5-benzoyl-2-(2-pyridylsulfinylméthyl)-benzimidazole

A une solution de 17,3g (0,05 mole) de 5-benzoyl-2-(2-pyridylthiométhyl)-benzimidazole dans 250 ml de dichlorométhane, refroidie à -15 ° C, on ajoute lentement 10,1g (0,05 mole) d'acide m-chloroperoxybenzoïque à 85%. On agite à une température inférieure à -10 ° C pendant 30 minutes et on ajoute 100 ml d'une solution de carbonate de sodium. On décante la phase organique, on lave avec du carbonate de sodium et de l'eau et puis on sèche ($Na_2SO_4$). Une fois filtré, on évapore le solvant et on recristallise le résidu dans l'acétone. On obtient 12,0g (66,5%) de 5-benzoyl-2-(2-pyridylsulfinylméthyl)-benzimidazole, de point de fusion 142-145 ° C.

Les données pour l'identification du produit se présentent dans les tableaux 3 et 4.

EXEMPLES 28 à 53

Les composés identifiés par les exemples 28 à 53 dans les tableaux 3 et 4 sont préparés par la même méthode de préparation que celle décrite à l'exemple 27.

TABLEAU 3

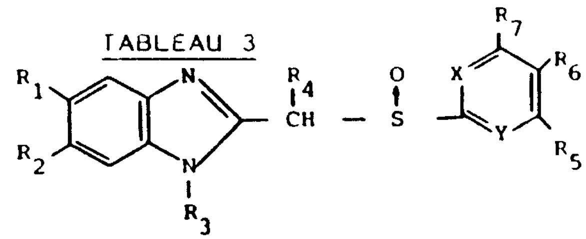

| Exemple n° | X | Y | $R_1$ | $R_2$ | $R_3$ | $R_4$ | $R_5$ | $R_6$ | $R_7$ | p.f. °C | IR (KBr) $cm^{-1}$ |
|---|---|---|---|---|---|---|---|---|---|---|---|
| 27 | CH | N | $C_6H_5CO-$ | H | H | H | H | H | H | 142-5 | 1660;1030 (Base) |
| 28 | CH | N | H | H | H | H | H | H | H | 162-3 | 1040 (Base) |
| 29 | CH | N | H | H | H | H | H | $NO_2$ | H | 173-6 | 1530;1360 1050 (Base) |
| 30 | CH | N | $CH_3$ | H | H | H | H | H | H | 157-9 | 1030 (Base) |
| 31 | CH | N | $CH_3$ | $CH_3$ | H | H | H | H | H | 198-9 | 1025 (Base) |
| 32 | CH | N | Cl | H | H | H | H | H | H | 176 | 1030 (Base) |
| 33 | N | N | H | H | H | H | $CH_3$ | H | $CH_3$ | 144-50 | 1050 (Base) |
| 34 | CH | N | $CF_3$ | H | H | H | H | H | H | 196-8 | 1030 (Base) |
| 35 | CH | N | $CH_3O$ | H | H | H | H | H | H | 109-16 | 1035 (Base) |
| 36/37 | CH | N | H | H | H | $CH_3$ | H | H | H | 131-4 | 1040;1050 (Base) |
| 38 | CH | N → O | H | H | H | H | H | H | H | 181-3 | 1045 (Base) |
| 39 | CH | N | H | H | H | H | H | H | H | 173-5 | 1035 (HI) |
| 40 | N | N | H | H | H | H | H | H | H | 140-5 | 1065 (Base) |

TABLEAU 3 (Continuation)

| Exemple nº | X | Y | $R_1$ | $R_2$ | $R_3$ | $R_4$ | $R_5$ | $R_6$ | $R_7$ | p.f. ºC | IR (KBr) $cm^{-1}$ |
|---|---|---|---|---|---|---|---|---|---|---|---|
| 41 | $C-CH_3$ | N | $C_6H_5CO-$ | H | H | H | H | $CH_3$ | H | 145-9 | 1655;1040 (Base) |
| 42 | $C-CH_3$ | N | $C_6H_5CO-$ | H | H | H | H | H | H | 156-160 | 1650;1040 (HCl) |
| 43 | $C-CH_3$ | N | H | H | H | H | H | H | H | 165-8 | 1020 (Base) |
| 44 | CH | N | $C_6H_5CO-$ | H | H | H | H | H | $CH_3$ | 157-161 | 1655;1045 (HCl) |
| 45 | CH | N | $CH_3OOC-$ | H | H | H | H | H | H | 180-1 | 1710;1040 (Base) |
| 46 | CH | N | H | H | H | H | H | H | $CH_3$ | 172-6 | 1055 (HCl) |
| 47 | CH | N | $CF_3$ | H | H | H | H | H | $CH_3$ | 180-2 | 1045;1035 (Base) |
| 48 | CH | N | $CH_3OOC-$ | H | H | H | H | H | $CH_3$ | 138-40 | 1710;1030 (Base) |
| 49 | CH | N | $CH_3O$ | H | H | H | H | H | $CH_3$ | 130-5 | 1060 (HCl) |
| 50 | CH | N | $CH_3CO$ | H | H | H | H | H | $CH_3$ | 148-50 | 1675;1035 (Base) |
| 51 | CH | N | $CH_3$ | H | H | H | H | H | $CH_3$ | 169-72 | 1060 (HCl) |
| 52 | CH | N | $CH_3$ | $CH_3$ | H | H | H | H | $CH_3$ | 170-1 | 1020 (Base) |
| 53 | CH | N | Cl | H | H | H | H | H | $CH_3$ | 146-9 | 1030 (Base) |

TABLEAU 4

| Exemple n° | $^1H$ RMN δ [ DMSO - $d_6$ ] |
|---|---|
| 27 | 12,80 (é,1H); 8,70 (d,1H); 8,13-7,40 (m,11H); 4,75 (d,1H); 4,42 (d,1H) |
| 28 | 12,50 (é,1H); 8,69 (d,1H); 8,10-7,70 (m,2H); 7,65-7,05 (m,5H); 4,77 (d,1H); 4,42 (d,1H) |
| 29 | 12,52 (é,1H); 9,50 (d,1H); 8,79 (q,1H); 7,95 (d,1H); 7,31 (m,4H); 4,79 (d,1H) 4,44 (d,1H) |
| 30 | 12,40 (é,1H); 8,69 (d,1H); 8,10-6,90 (m,6H); 4,70 (d,1H); 4,35 (d,1H); 2,38 (s,3H) |
| 31 | 12,0 (é,1H); 8,70 (q,1H); 8,02 (m,1H); 7,79 (q,1H); 7,51 (m,1H); 7,27 (s,2H) 4,64 (d,1H); 4,31 (d,1H); 2,30 (s,6H) |
| 32 | 12,65 (é,1H); 8,69 (d,1H); 8,10-7,45 (m,5H); 7,17 (q,1H); 4,70 (d,1H); 4,36 (d,1H) |
| 33 | 12,46 (é,1H); 7,53 (m,2H); 7,41 (s,1H); 7,19 (m,2H); 4,66 (d,1H); 4,49 (d,1H); 2,46 (s,6H) |
| 34 | 12,89 (é,1H); 8,72 (d,1H); 8,20-7,40 (m,6H); 4,75 (d,1H); 4,41 (d,1H) |
| 35 | 12,33 (é,1H); 8,69 (d,1H); 8,12-6,74 (m,6H); 4,67 (d,1H); 4,32 (d,1H); 3,76 (s,3H) |
| 36 | 12,30 (é,1H); 8,61 (d,1H); 7,95-7,00 (m,7H); 4,62 (q,1H); 1,74 (d,3H) |
| 37 | 12,30 (é,1H); 8,55 (d,1H); 7,90-7,28 (m,7H); 4,95 (q,1H); 1,58 (d,3H) |
| 38 | 12,41 (é,1H); 8,43 (d,1H); 7,68-7,00 (m,7H); 4,94 (d,1H); 4,69 (d,1H) |
| 39 | 11,20 (é,2H); 8,72 (d,1H); 8,10-7,83 (m,2H); 7,76-7,25 (m,5H); 4,91 (d,1H); 4,54 (d,1H) |
| 40 | 12,58 (é,1H); 8,98 (d,2H); 7,71 (d,1H); 7,58-7,10 (m,4H); 4,73 (d,1H); 4,51 (d,1H) |

TABLEAU 4 (Continuation)

| Exemple n° | $^1$H RMN δ [DMSO - $d_6$] |
| --- | --- |
| 41 | 9,47 (é,1H); 8,39 (s,1H); 7,92 (s,1H); 7,84-7,40 (m,8H); 4,78 (d,1H); 4,63 (d,1H); 2,37 (s,3H); 2,26 (s,3H) |
| 42 | 12,94 (é,1H); 8,57 (d,1H); 7,93 (s,1H); 7,67 (m,9H); 4,77 (d,1H); 4,66 (d,1H); 2,43 (s,3H) |
| 43 | 8,58 (dd,1H); 7,74 (é,1H); 7,71-7,35 (m,4H); 7,16 (m,2H); 4,73 (d,1H); 4,59 (d,1H); 2,38 (s,3H) |
| 44 | 8,54 (d,1H); 7,90 (d,1H); 7,63 (m,9H); 7,35 (d,1H); 4,73 (d,1H); 4,39 (d,1H); 2,35 (s,3H) |
| 45 | 12,81 (é,1H); 8,70 (d,1H); 8,34-7,54 (m,6H); 4,77 (d,1H); 4,44 (d,1H); 3,87 (s,3H) |
| 46 | 12,52 (é,1H); 8,54 (d,1H); 7,58 (m,3H); 7,23 (m,3H); 4,72 (d,1H); 4,37 (d,1H); 2,34 (s,3H) |
| 47 | 9,82 (é,1H); 8,53 (d,1H); 7,89 (s,1H); 7,72 (d,1H); 7,58 (s,1H); 7,47 (d,1H); 7,34 (d,1H); 4,74 (d,1H); 4,41 (d,1H); 2,33 (s,3H) |
| 48 | 12,84 (é,1H); 8,54 (d,1H); 8,16 (s,1H); 7,72 (m,3H); 7,33 (d,1H); 4,74 (d,1H); 4,41 (d,1H); 3,87 (s,3H); 2,34 (s,3H) |
| 49 | 12,24 (é,1H); 8,55 (d,1H); 7,63 (s,1H); 7,37 (m,2H); 7,03 (s,1H); 6,82 (d,1H); 4,63 (d,1H); 4,28 (d,1H); 3,78 (s,3H); 2,38 (s,3H) |
| 50 | 12,85 (é,1H); 8,54 (d,1H); 8,18 (s,1H); 7,83 (d,1H); 7,61 (s,1H); 7,56 (d,1H); 7,35 (d,1H); 4,74 (d,1H); 4,40 (d,1H); 2,62 (s,3H); 2,35 (s,3H) |
| 51 | 9,95 (é,1H); 8,56 (d,1H); 7,64 (s,1H); 7,37 (m,3H); 7,00 (d,1H); 4,64 (d,1H); 4,28 (d,1H); 2,44 (s,3H); 2,38 (s,3H) |
| 52 | 12,33 (é,1H); 8,56 (d,1H); 7,65 (s,1H); 7,36 (d,1H); 7,29 (s,2H); 4,61 (d,1H); 4,26 (d,1H); 2,40 (s,3H); 2,31 (s,6H) |
| 53 | 12,63 (é,1H); 8,50 (d,1H); 7,58 (m,2H); 7,49 (s,1H); 7,29 (d,1H); 7,16 (d,1H); 4,70 (d,1H); 4,36 (d,1H); 2,32 (s,3H) |

EXEMPLE 54

Méthode E : Préparation de 5-chloro-2-(2-pyridylsulfonylméthyl)-benzimidazole

On ajoute 5,7g (0,028 mole) d'acide m-chloroperoxybenzoïque à 85% à une solution de 2,76g (0,01 mole) de 5-chloro-2-(2-pyridylthiométhyl)-benzimidazole dans 75 ml de chloroforme. On agite à température ambiante pendant 4 heures et on ajoute 50 ml d'une solution de carbonate de sodium. On décante la phase organique, on lave avec du bicarbonate de sodium et de l'eau et on sèche ($Na_2SO_4$). Une fois filtré, on évapore le solvant, on recristallise le résidu dans l'acétate d'éthyle et on obtient 2,55g (83%) de 5-chloro-2-(2-pyridylsulfonylméthyl)-benzimidazole de point de fusion 183-186 ° C.

Les données pour l'identification du produit se présentent dans les tableaux 5 et 6.

EXEMPLES 55 à 58

Les composés identifiés par les exemples 55 à 58 dans les tableaux 5 et 6 sont préparés par la même méthode de préparation que celle décrite dans l'exemple 54.

TABLEAU 5

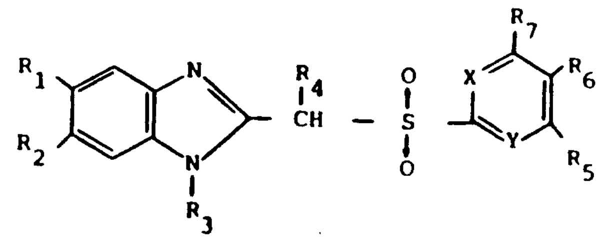

| Exemple n° | X | Y | $R_1$ | $R_2$ | $R_3$ | $R_4$ | $R_5$ | $R_6$ | $R_7$ | p.f. °C | IR (KBr) $cm^{-1}$ |
|---|---|---|---|---|---|---|---|---|---|---|---|
| 54 | CH | N | Cl | H | H | H | H | H | H | 183-6 | 1305;1165 |
| 55 | CH | N | H | H | H | H | H | H | H | 213 | 1330;1170 |
| 56 | CH | N | $CH_3$ | H | H | H | H | H | H | 171-3 | 1320;1165; 1160 |
| 57 | CH | N | $CH_3$ | $CH_3$ | H | H | H | H | H | 212-4 | 1305;1165 |
| 58 | CH | N | H | H | H | $CH_3$ | H | H | H | 191-5 | 1325;1165 |

TABLEAU 6

| Exemple n° | $^1H$ RMN $_\delta$[ DMSO $d_6$ ] |
|---|---|
| 54 | 12,30 (é,1H); 8,84 (d,1H); 8,20-7,48 (m,5H); 7,19 (d,1H); 5,14 (s,2H) |
| 55 | 12,63 (é,1H); 8,88 (d,1H); 8,25-7,70 (m,3H); 7,49 (m,2H); 7,17 (m,2H); 5,10 (s,2H) |
| 56 | 12,42 (é,1H); 8,84 (d,1H); 8,20-7,60 (m,3H); 7,35 (m,2H); 6,97 (d,1H); 5,07 (s,2H); 2,38 (s,3H) |
| 57 | 12,24 (é,1H); 8,84 (d,1H); 8,17-7,65 (m,3H); 7,25 (s,2H); 5,02 (s,2H); 2,28 (s,6H) |
| 58 | 12,37 (é,1H); 8,60 (d,1H); 8,14-7,00 (m,7H); 5,28 (q,1H); 1,75 (d,3H) |

EXEMPLE 59

Méthode F : Préparation de 5-nitro-2-(2-pyridylsulfinylméthyl)-benzimidazole

A une solution de 2,57g (0,01 mole) de 2-(2-pyridyl sulfinylméthyl)-benzimidazole dans 20 ml d'acide sulfurique concentré, refroidie à -5° C, on ajoute un mélange, refroidi également, de 2 ml d'acide nitrique à 65% et 3 ml d'acide sulfurique concentré, de façon que la température ne dépasse pas 0° C. Une fois l'addition terminée, on maintient une heure sous agitation et on verse sur de la glace.

On neutralise avec de l'hydroxyde de sodium jusqu'à un pH de 7,1 à 7,2, on filtre et on lave avec de l'eau. Une fois sec on obtient 2,93g (97%) de 5-nitro-2-(2-pyridylsulfinylméthyl)-benzimidazole, de point de fusion 207-209° C.

Les données pour l'identification du produit se présentent dans les tableaux 7 et 8.

EXEMPLE 19

Méthode G : Préparation de 5-méthoxycarbonyl-2-(4-méthyl-2-pyridylthiométhyl)-benzimidazole

On chauffe à reflux pendant 6 heures une solution de 5,98 g d'acide 5-carboxy-2-(4-méthyl-2-pyridylthiométhyl)-benzimidazole (0,02 mole) dans 100 ml de méthanol saturé en acide chlorhydrique. On évapore le méthanol et on reprend avec 25 ml d'éthanol. De cette solution cristallisent 5,82 g (93%) de 5-méthoxycarbonyl-2-(4-méthyl-2-pyridylthiométhyl) -benzimidazole hydrochloride, de point de fusion 121-4° C. Les données pour l'identification du produit se présentent dans les tableaux 1 et 2.

Le composé identifié par l'exemple 20 dans les tableaux 1 et 2 est préparé par la même méthode de préparation que celle décrite à l'exemple 19.

EXEMPLES 60 et 61

Méthode H : Préparation de 5-benzoyl-1-méthyl-2-(2-pyridylsulfinyl méthyl)-benzimidazole et Préparation de 6-benzoyl-1-méthyl-2-(2-pyridylsulfinyl méthyl)-benzimidazole

Un mélange de 3,61g de 5-benzoyl-2-(2-pyridylsulfinylméthyl)-benzimidazole (0,01 mole), 0,40g d'hydroxyde de sodium (0,01 mole) et 2 ml de sulfate de diméthyle (0,02 mole) dans 50 ml d'éthanol et 5 ml d'eau, est maintenu à reflux pendant 3 heures. On évapore l'éthanol, on dilue avec de l'eau et on extrait avec du chloroforme. On lave la phase organique avec de l'eau, on sèche et on évapore. On chromatographie le résidu avec du gel de silice en utilisant comme éluant le chloroforme/méthanol (99:1) et postérieurement le chloroforme/méthanol (95:5). Par cristallisation fractionnée dans l'acétate d'éthyle de la dernière fraction, on obtient 0,47g (12,5%) de 5-benzoyl-1-méthyl-2-(2-pyridylsulfinylméthyl)-benzimidazole, de point de fusion 174-177° C et 1,5g (40%) de 6-benzoyl-1-méthyl-2-(2-pyridylsulfinylméthyl)-benzimidazole, de point de fusion 181-183° C.

Les données pour l'identification des produits correspondants aux exemples 60 et 61 se présentent dans les tableaux 7 et 8.

EXEMPLES 62 et 63

Méthode I : Préparation de 5-benzoyl-1-éthoxycarbonyl-2-(2-pyridylsulfinylméthyl)-benzimidazole

A une solution de 3,61g de 5-benzoyl-2-(2-pyridylsulfinylméthyl)-benzimidazole (0,01 mole) et 1,41g de

triéthylamine (0,014 mole) dans 60 ml de chlorure de méthylène, on ajoute 1,54g de chloroformiate d'éthyle (0,014 mole). On agite pendant 24 heures à température ambiante et on lave avec une solution diluée d'hydroxyde de sodium, puis avec de l'eau et on sèche ($Na_2SO_4$). On évapore à sec la phase organique et on chromatographie avec du gel de silice en utilisant comme éluant acétate d'éthyle, et on obtient les deux isomères: 1,2g (27,7%) de 5-benzoyl-1-éthoxycarbonyl-2-(2-pyridylsulfinylméthyl)-benzimidazole et 1,6g (36,9%) de 6-benzoyl-1-éthoxycarbonyl-2-(2-pyridylsulfinylméthyl)benzimidazole.

Les données pour l'identification des produits correspondants aux exemples 62 et 63 se présentent dans les tableaux 7 et 8.

EXEMPLE 64

Le composé identifié par l'exemple 64 dans les tableaux 7 et 8 est préparé par la même méthode de préparation que celle décrite dans les exemples 60 et 61.

TABLEAU 7

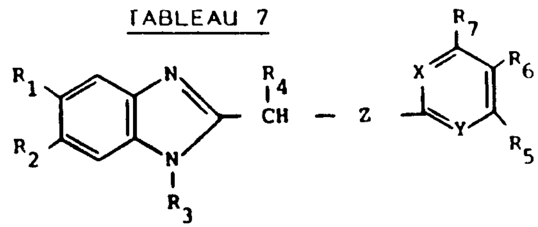

| Exemple n° | X | Y | Z | $R_1$ | $R_2$ | $R_3$ | $R_4$ | $R_5$ | $R_6$ | $R_7$ | Méthode | p.f. °C | IR (KBr) $cm^{-1}$ |
|---|---|---|---|---|---|---|---|---|---|---|---|---|---|
| 59 | CH | N | S→O | $NO_2$ | H | H | H | H | H | H | F | 207-9 | 1515;1345; 1040 |
| 60 | CH | N | S→O | $C_6H_5CO-$ | H | $CH_3$ | H | H | H | H | H | 174-7 | 1660;1050 |
| 61 | CH | N | S→O | H | $C_6H_5CO-$ | $CH_3$ | H | H | H | H | H | 181-3 | 1650; 1048 |
| 62 | CH | N | S→O | $C_6H_5CO-$ | H | $CO_2CH_2CH_3$ | H | H | H | H | I | | 1760;1660; 1055 |
| 63 | CH | N | S→O | H | $C_6H_5CO-$ | $CO_2CH_2CH_3$ | H | H | H | H | I | 142-5 | 1760;1650; 1050 |
| 64 | CH | N | S→O | H | H | $CH_2CH_3$ | H | H | H | H | H | 102-5 | 1040 |

TABLEAU 8

| Exemple n° | $^1H$ RMN $_\delta$ [ DMSO - $d_6$ ] |
|---|---|
| 59 | 13,15 (é,1H); 8,69 (d,1H); 8,41 (s,1H); 8,04 (m,2H); 7,64 (m,3H); 4,78 (d,1H); 4,44 (d,1H) |
| 60 | 8,70 (d,1H); 8,15-7,50 (m,11H); 4,93 (d,1H); 4,62 (d,1H); 3,85 (s,3H) |
| 61 | 8,71 (d,1H); 8,10-7,50 (m,11H); 4,96 (d,1H) 4,63 (d,1H); 3,83 (s,3H) |
| 62 | 8,64 (d,1H); 8,29-7,48 (m,11H); 5,17 (d,1H); 4,71 (d,1H); 4,53 (q,2H); 1,46 (t,3H) |
| 63 | 8,64 (d,1H); 8,32-7,48 (m,11H); 5,18 (d,1H); 4,75 (d,1H); 4,47 (q,2H); 1,34 (t,3H) |
| 64 | 8,71 (d,1H); 8,15-7,16 (m,7H); 4,83 (d,1H); 4,52 (d,1H); 4,31 (q,2H); 1,34 (t,3H) |

Activité inhibitrice de la sécrétion acide gastrique

Méthode de Shay |Shay, H.; Komarov, S.A.; Fels, S.S.; Merange, D.; Grvenstein, M.; Siplet, H.: Gastroenterology, 5, 43 (1945) - Visscher, F.E.; Seay, P.H.; Taxelaar, A.P.; Veldkamp, W.; Vander Brook, M.J.: J.Pharmac. Exp.Ther., 110, 188 (1954) - "Animal Experiments in Pharmacological Analysis", F.R. Domer; C.C. Thomas Pub, Springfield, Illinois, USA, 1970, p. 140|.

Dans ce test on utilise des rats Wistar, mâles, de 200 à 250 grammes de poids, que l'on maintient à jeun depuis le jour antérieur à celui de l'essai, avec accès libre à l'eau. On utilise des lots de 4 animaux chacun, au minimum.

On anesthésie les rats avec de l'éther éthylique, on leur pratique une laparotomie et on leur ligature le pylore, puis on effectue la couture de l'incision abdominale. L'administration des produits, avec le véhicule pour le lot témoin, s'effectue par voie intraduodénale (i.d.) avant de coudre l'incision abdominale. La dose administrée pour le premier essai est de 40 mg/kg et on détermine également dans un deuxième essai la dose efficace cinquante (DE-50) par voie i.d. Le véhicule utilisé est la gomme arabique à 5% p/v dans l'eau bidistillée.

Deux heures après la ligature du pylore, on sacrifie les rats par anesthésie prolongée avec de l'éther éthylique et on mesure le volume du suc gastrique, on détermine l'acidité totale moyennant un pH-mètre muni d'une burette automatique. Pour chaque produit et pour chaque dose essayés, on détermine le pourcentage d'inhibition de la sécrétion acide gastrique, par rapport au lot témoin.

A titre d'exemples, on résume les résultats obtenus pour quelques uns des dérivés de la présente invention dans le tableau 9.

TABLEAU 9

| Inhibition de la sécrétion acide gastrique chez le rat Shay | | |
|---|---|---|
| Produit | Pourcentage d'inhibition de la sécrétion acide gastrique Dose = 40 mg/kg, voie i.d.) | DE-50 (mg/kg, voie i.d.) |
| Exemple 1 | 98 | 13 |
| Exemple 3 | 88 | 16 |
| Exemple 4 | 74 | 21 |
| Exemple 6 | 92 | 19 |
| Exemple 7 | 95 | 19 |
| Exemple 10 | 93 | 14 |
| Exemple 27 | 95 | 7 |
| Exemple 28 | 90 | 8 |
| Exemple 29 | 90 | 4 |
| Exemple 31 | 96 | 15 |
| Exemple 32 | 92 | 17 |
| Exemple 41 | 80 | 10 |
| Exemple 44 | 66 | 10 |
| Exemple 54 | 70 | 24 |
| Exemple 60 | 75 | 21 |
| Exemple 63 | 81 | 15 |

Toxicité aiguë chez la souris

Méthode de Litchfield et Wilcoxon (Litchfield, J.T. et Wilcoxon, E.J., J. Pharmacol. Exp. Therap., 96, 19-113 (1949)).

On administre le produit par voie orale en suspension dans la gomme arabique à 5% dans l'eau bidistillée. Le volume administré est de 10 ml/kg. On calcule selon la méthode citée la dose léthale cinquante (DL-50).

A titre d'exemples, on résume les résultats obtenus pour quelque uns des dérivés de la présente invention dans le tableau 10.

TABLEAU 10

| produit | Sexe | DL-50 (mg/kg) voie orale |
|---|---|---|
| Exemple 1 | ♂ | 3200 |
| | ♀ | 2700 |
| Exemple 3 | ♂ | > 3200 |
| | ♀ | > 3200 |
| Exemple 27 | ♂ | 3200 |
| | ♀ | 3200 |
| Exemple 28 | ♂ | 3200 |
| | ♀ | 3200 |
| Exemple 29 | ♂ | 2400 |
| | ♀ | 2400 |
| Exemple 31 | ♂ | > 6400 |
| | ♀ | > 6400 |

En thérapeutique humaine, la dose d'administration des dérivés de la présente invention est bien sûr fonction de la gravité de l'affection à traiter, elle sera généralement comprise entre environ 30 et environ 60 mg/jour. Les dérivés de l'invention seront par exemple administrés sous la forme de comprimés ou d'ampoules injectables.

On indiquera ci-après, à titre d'exemples, deux formes galéniques particulières des dérivés objet de la présente invention.

Exemple de formule par comprimé

| Comprimés de 30 mg | |
|---|---|
| Exemple 27 | 0,030 g |
| Lactose | 0,0342g |
| Amidon | 0,030 g |
| Polyvinylpyrrolidone | 0,006 g |
| Cellulose microcristalline | 0,018 g |
| Silice colloïdal | 0,0012g |
| Stéarate de magnésium | 0,0006g |
| | 0,120 g |

| Comprimés de 60 mg | |
|---|---|
| Exemple 27 | 0,060 g |
| Lactose | 0,0684g |
| Amidon | 0,060 g |
| Polyvinylpyrrolidone | 0,012 g |
| Cellulose microcristalline | 0,036 g |
| Silice colloïdal | 0,0024g |
| Stéarate de magnésium | 0,0012g |
| | 0,240 g |

Exemple de formule par ampoule injectable

| Injectables 6 mg/ml | |
|---|---|
| Exemple 27 | 0,030 g |
| Chlorure de sodium q.s. | 0,050 g |
| Acide ascorbique | 0,005 g |
| Eau p. injection q.s.p | 5 ml |

## Revendications

**Revendications pour les Etats contractants suivants : BE, CH, DE, FR, GB, IT, LI, LU, NL, SE**

**1.** Dérivés de 2-alkylbenzimidazole répondant à la formule générale I

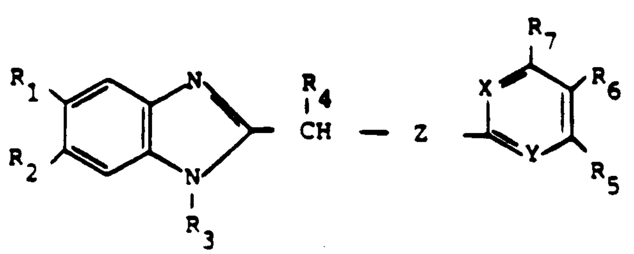

I

ainsi que leurs sels thérapeutiquement acceptables, dans laquelle:

X représente un atome d'azote (N) ou un atome de carbone lié à un autre radical $R_8$ ($C-R_8$);

Y représente un atome d'azote (N) ou un groupe N - oxyde ($N \rightarrow O$);

Z représente un atome de soufre (S), un groupe sulfinyle ($S \rightarrow O$) ou un groupe sulfonyle ($O \leftarrow S \rightarrow O$);

$R_1$ et $R_2$ identiques ou différents, représentent un atome d'hydrogène, un halogène, un radical alkyle inférieur linéaire ou ramifié en $C_1$ à $C_4$, un groupe nitro ($NO_2$), un groupe trifluorométhyle ($CF_3$), un radical alkoxy inférieur en $C_1$ à $C_4$, un radical méthylcarbonyle, méthoxycarbonyle, benzoyle ;

$R_3$ représente un atome d'hydrogène, un radical alkyle inférieur en $C_1$ à $C_4$, un radical carbonyle lié à un autre radical $R_9$

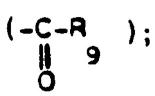

$R_4$ représente un atome d'hydrogène, un radical alkyle inférieur en $C_1$ à $C_4$ ;

$R_5$ représente un atome d'hydrogène, un radical méthyle, un radical hydroxy;
$R_6$ représente un atome d'hydrogène, un radical méthyle, un radical nitro ($NO_2$);
$R_7$ représente un atome d'hydrogène, un radical alkyle inférieur en $C_1$ à $C_4$;
$R_8$ représente un atome d'hydrogène, un radical méthyle;
$R_9$ représente un radical alkoxy;

à l'exception toutefois du composé de formule I, dans laquelle:
X représente CH,
Y représente N,
Z représente S, et
$R_1$ à $R_7$ représentent H.

2. Les composés répondant à la formule générale I, selon la revendication 1, sélectionnés dans le groupe suivant :
- 2-(2-benzimidazolylméthylthio)-pyrimidine
- 2-(2-benzimidazolylméthylthio)-4-hydroxy-6-(n-propyl)-pyrimidine
- 2-(2-benzimidazolylméthylthio)-4,6-diméthylpyrimidine
- 2-(5-nitro-2-pyridylthiométhyl)-benzimidazole
- 5-méthyl-2-(2-pyridylthiométhyl)-benzimidazole
- 5,6-diméthyl-2-(2-pyridylthiométhyl)-benzimidazole
- 2-(1-oxyde-2-pyridylthiométhyl)-benzimidazole
- 5-chloro-2-(2-pyridylthiométhyl)-benzimidazole
- 5-trifluorométhyl-2-(2-pyridylthiométhyl)-benzimidazole
- 5-benzoyl-2-(2-pyridylthiométhyl)-benzimidazole
- 5-méthoxy-2-(2-pyridylthiométhyl)-benzimidazole
- 2-[2-pyridylthio-(méthyl)-méthyl]-benzimidazole
- 5-benzoyl-2-(3,5-diméthyl-2-pyridylthiométhyl)-benzimidazole
- 5-benzoyl-2-(3-méthyl-2-pyridylthiométhyl)-benzimidazole
- 2-(3-méthyl-2-pyridylthiométhyl)-benzimidazole
- 2-(4-méthyl-2-pyridylthiométhyl)-benzimidazole
- 5-trifluorométhyl-2-(4-méthyl-2-pyridylthiométhyl)-benzimidazole
- 5-méthoxycarbonyl -2-(4-méthyl-2-pyridylthiométhyl)-benzimidazole
- 5-méthoxycarbonyl -2-(2-pyridylthiométhyl)-benzimidazole
- 5-benzoyl-2-(4-méthyl-2-pyridylthiométhyl)-benzimidazole
- 5-méthoxy-2-(4-méthyl-2-pyridylthiométhyl)-benzimidazole
- 5-acetyl-2-(4-méthyl-2-pyridylthiométhyl)-benzimidazole
- 5-méthyl-2-(4-méthyl-2-pyridylthiométhyl)-benzimidazole
- 5,6-diméthyl-2-(4-méthyl-2-pyridylthiométhyl)-benzimidazole
- 5-chloro-2-(4-méthyl-2-pyridylthiométhyl)-benzimidazole

3. Les composés répondant à la formule générale I, selon la revendication 1, sélectionnés dans le groupe suivant :
- 5-benzoyl-2-(2-pyridylsulfinylméthyl)-benzimidazole
- 2-(2-pyridylsulfinylméthyl)-benzimidazole
- 2-(5-nitro-2-pyridylsulfinylméthyl)-benzimidazole
- 5-méthyl-2-(pyridylsulfinylméthyl)-benzimidazole
- 5,6-diméthyl-2-(2-pyridylsulfinlyméthyl)-benzimidazole
- 5-chloro-2-(2-pyridylsulfinlyméthyl)-benzimidazole
- 2-(2-benzimidazolylméthylsulfinyl)-4,6-diméthylpyrimidine
- 5-trifluorométhyl-2-(2-pyridylsulfinylméthyl)-benzimidazole
- 5-méthoxy-2-(2-pyridylsulfinylméthyl)-benzimidazole
- érythro-2-[2-pyridylsulfinly-(méthyl)-méthyl]-benzimidazole
- thréo-2-[2-pyridylsulfinyl-(méthyl)-méthyl]-benzimidazole
- 2-(1-oxyde-2-pyridylsulfinylméthyl)-benzimidazole
- 2-(2-benzimidazolylméthylsulfinyl)-pyrimidine
- 5-benzoyl-2-(3,5-diméthyl-2-pyridylsulfinylméthyl)-benzimidazole
- 5-benzoyl-2-(3-méthyl-2-pyridylsulfinylméthyl)-benzimidazole
- 2-(3-méthyl-2-pyridylsulfinylméthyl)-benzimidazole
- 5-benzoyl-2-(4-méthyl-2-pyridylsulfinylméthyl)-benzimidazole

- 5-méthoxycarbonyl -2-(2-pyridylsulfinylméthyl)-benzimidazole
- 2-(4-méthyl-2-pyridylsulfinylméthyl)-benzimidazole
- 5-trifluorométhyl-2-(4-méthyl-2-pyridylsulfinylméthyl)-benzimidazole,
- 5-méthoxycarbonyl -2-(4-méthyl-2-pyridylsulfinylméthyl)-benzimidazole
- 5-méthoxy-2-(4-méthyl-2-pyridylsulfinylméthyl)-benzimidazole
- 5-acetyl-2-(4-méthyl-2-pyridylsulfinylméthyl)-benzimidazole
- 5-méthyl-2-(4-méthyl-2-pyrdidylsulfinylméthyl)-benzimidazole
- 5,6-diméthyl-2-(4-méthyl-2-pyridylsulfinylméthyl)-benzimidazole
- 5-chloro-2-(4-méthyl-2-pyridylsulfinylméthyl)-benzimidazole

**4.** Les composés répondant à la formule générale I, selon la revendication 1, sélectionnés dans le groupe sui-vant :
- 5-chloro-2-(2-pyridylsulfonylméthyl)-benzimidazole
- 2-(2-pyridylsulfonylméthyl)-benzimidazole
- 5-méthyl-2-(2-pyridylsulfonylméthyl)-benzimidazole
- 5,6-diméthyl-2-(2-pyridylsulfonylméthyl)-benzimidazole
- 2-[2-pyridylsulfonyl-(méthyl)-méthyl]-benzimidazole

**5.** Les composés répondant à la formule générale I, selon la revendication 1, sélectionnés dans le groupe suivant :
- 5-nitro-2-(2-pyridylsulfinylmethyl)benzimidazole
- 5-benzoyl-1-méthyl-2-(2-pyridylsulfinylméthyl)-benzimidazole
- 6-benzoyl-1-méthyl-2-(2-pyridylsulfinylméthyl)-benzimidazole
- 5-benzoyl-1-éthoxycarbonyl(2-pyridylsulfinylméthyl)-benzimidazole
- 6-benzoyl-1-éthoxycarbonyl(2-pyridylsulfinylméthyl)-benzimidazole
- 1-éthyl-2-(2-pyridylsulfinylméthyl)-benzimidazole.

**6.** Procédé de préparation des composés selon l'une des revendications 1 à 5, caractérisé par la mise en oeuvre d'au moins l'une des opérations suivantes :

A) Par réaction d'un composé de formule générale II

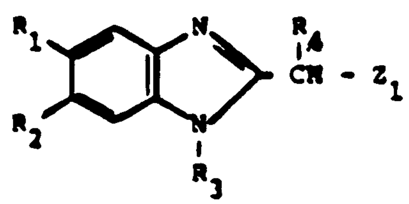

II

avec un composé de formule générale III

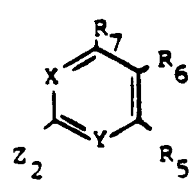

III

dans lesquelles les radicaux X, Y et $R_1$ à $R_7$ ont les significations données à la revendication 1, et l'un des deux radicaux $Z_1$ et $Z_2$ est constitué par le radical -SH et l'autre est un groupe partant notamment choisi parmi les halogènes, de préférence le fluor, le chlore ou le brome ; des radicaux formés par des groupes estérifiés et qui soient réactifs notamment acétyloxy, tosyloxy ou mésyloxy ; ou encore des groupes alkylthio ou alkylsulfinyle, par exemple méthylthio ou méthylsulfinyle ; ou bien

B) Par réaction d'un composé de formule générale II avec un composé de formule générale III, dans lesquelles : X et $R_1$ à $R_7$ ont les significations mentionnées dans la revendication 1, Y représente un

groupe N-oxyde (N → O)
$Z_1$ est constitué par le radical -SH et $Z_2$ représente un atome d'hydrogène,
on prépare un composé de formule générale I dans laquelle X et $R_1$ à $R_7$ ont les significations données à la revendication 1,
Y représente un atome d'azote et Z représente un atome de soufre ; ou bien
C) Par réaction d'un composé de formule générale IV

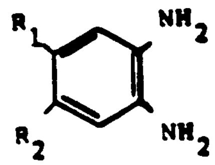

IV

avec un composé de formule générale V

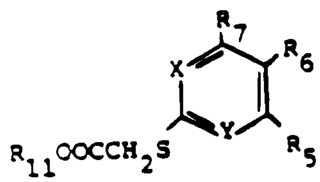

V

dans lesquelles les radicaux X, Y, $R_1$, $R_2$, $R_5$, $R_6$ et $R_7$ ont les significations données à la revendication 1 et $R_{11}$ représente un atome d'hydrogène ou un radical alkyle inférieur, tel que méthyle ou éthyle,
on prépare un composé de formule générale I dans laquelle X, Y, $R_1$, $R_2$, $R_5$, $R_6$ et $R_7$ ont les significations données à la revendication 1, $R_3$ et $R_4$ représentent un atome d'hydrogène et Z représente un atome de soufre, ou bien
D) Par oxydation, avec une quantite stoechiométrique d'un agent oxydant, des composés de formule générale I dans laquelle X, Y et $R_1$ à $R_7$ ont les significations données à la revendication 1 et Z représente un atome de soufre, on prépare des composés de formule générale I dans laquelle X, Y et $R_1$ à $R_7$ ont les significations données à la revendication 1 et Z représente un groupe sulfinyle (S → O);
Ledit agent oxydant étant de préférence choisi parmi le peroxyde d'hydrogène, les peracides tels que l'acide m-chloroperbenzoïque, l'acide nitrique, le métapériodate de sodium, l'acide chromique, le dioxyde de manganèse, le chlore, le brome ou le chlorure de sulfuryle ; ou bien
E) Par oxydation, avec une quantité d'un agent oxydant deux à trois fois supérieure à la quantité stoechiométrique des composés de formule générale I dans laquelle X, Y et
$R_1$ à $R_7$ ont les significations données à la revendication 1 et Z représente un atome de soufre,
on prépare des composés de formule générale I dans laquelle X, Y et $R_1$ à $R_7$ ont les significations données à la revendication 1 et Z représente un groupe sulfonyle (O ← S → O) ;
Ledit agent oxydant étant de préférence choisi parmi le peroxyde d'hydrogène, les peracides tels que l'acide m-chloroperbenzoïque, l'acide nitrique, le métapériodate de sodium, l'acide chromique, le dioxyde de manganèse, le chlore, le brome ou le chlorure de sulfuryle.

**7.** Procédé de préparation des composés sèlon l'une des revendications 1 à 5, caractérisé par la mise en oeuvre d'au moins l'une des opérations suivantes :
F) Par nitration, dans un mélange d'acide sulfurique et nitrique, des composés de formule générale I dans laquelle $R_2$ et $R_3$ représentent des atomes d'hydrogène et X, Y, Z, $R_1$, $R_4$ à $R_7$ ont les significations données à la revendication 1, on prépare des composés de formule générale I dans laquelle X, Y, Z, $R_1$ et $R_4$ à $R_7$ ont les significations données à la revendication 1, $R_2$ représente un groupe nitro($NO_2$) et $R_3$ représente un atome d'hydrogène, ou bien
G) Pour la préparation des composés de formule générale I où $R_1$ ou $R_2$ est méthoxycarbonyle-($COOCH_3$) $R_3$ représente un atome d'hydrogène et $R_2$ ou $R_1$, X, Y, Z et $R_4$ à $R_7$ ont les significations données à la revendication 1,

on fait réagir un composé de formule générale I dans laquelle $R_1$ ou $R_2$ représente un radical carboxyle (COOH), $R_3$ représente un atome d'hydrogène, et $R_2$ ou $R_1$, X, Y, Z et $R_4$ à $R_7$ ont les significations données à la revendication 1, avec du méthanol, ou bien

H) Pour la préparation des composés de formule générale I dans laquelle $R_3$ représente un radical alkyle inférieur en $C_1$ à $C_4$ et X, Y, Z, $R_1$, $R_2$ et $R_4$ à $R_7$ ont les significations données à la revendication 1,

on fait réagir un composé de formule générale I dans laquelle $R_3$ représente un atome d'hydrogène et X, Y, Z, $R_1$, $R_2$ et $R_4$ à $R_7$ ont les significations données à la revendication 1, avec un agent alkylant, tel que sulfate de diméthyle, diméthylformamide diméthylacétal ou un halogénure d'alkyle dans lequel les halogènes préférés sont le chlore, le brome et l'iode, ou bien

I) Pour la préparation des composés de formule générale I dans laquelle $R_3$ représente un radical carbonyle lié à un autre radical $R_9$

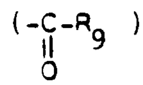

et X, Y, Z, $R_1$, $R_2$ et $R_4$ à $R_9$ ont les significations données à la revendication 1,

on fait réagir un composé de formule générale I dans laquelle $R_3$ représente un atome d'hydrogène et X, Y, Z, $R_1$, $R_2$ et $R_4$ à $R_7$ ont les significations données à la revendication 1, avec un composé de formule générale VI

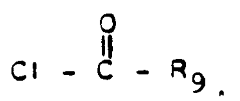

VI

dans laquelle $R_9$ a la signification donnée à la revendication 1.

8. A titre de médicaments, les dérivés de formule générale I et leurs sels thérapeutiquement acceptables selon l'une des revendications 1 à 5 , en particulier à titre de médicaments destinés au traitement des maladies gastro-intestinales principalement indiqués comme inhibiteurs de la sécrétion d'acide gastrique et comme agents cytoprotecteurs.

9. Compositions pharmaceutiques, caractérisées par le fait qu'elles contiennent, outre un support pharmaceutiquement acceptable, au moins un dérivés de formule générale I ou l'un de leurs sels physiologiquement acceptables, selon l'une des revendications 1 à 5.

10. Utilisation des dérivés de formule générale I et de leurs sels physiologiquement acceptables selon l'une des revendications 1 à 5 , pour la fabrication de médicaments destinés au traitement des maladies gastro-intestinales, en particulier pour la fabrication d'agents inhibiteurs de la sécrétion d'acide gastrique et d'agents cytoprotecteurs.

**Revendications pour les Etats contractants suivants : AT, GR**

1. Procédé de préparation des dérivés de 2-alkylbenzimidazole répondant à la formule générale I

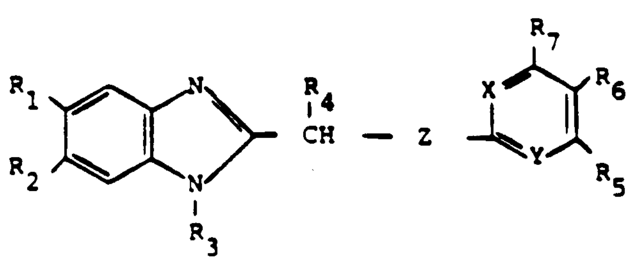

I

ainsi que leurs sels thérapeutiquement acceptables, dans laquelle:

X représente un atome d'azote (N) ou un atome de carbone lié à un autre radical $R_8$ (C-$R_8$);

Y représente un atome d'azote (N) ou un groupe N - oxyde (N → O);

Z représente un atome de soufre (S), un groupe sulfinyle (S → O) ou un groupe sulfonyle (O ← S → O);

$R_1$ et $R_2$ identiques ou différents, représentent un atome d'hydrogène, un halogène, un radical alkyle inférieur linéaire ou ramifié en $C_1$ à $C_4$, un groupe nitro ($NO_2$), un groupe trifluorométhyle ($CF_3$), un radical alkoxy en $C_1$ à $C_4$, un radical méthylcarbonyle, méthoxycarbonyle, benzoyle ;

$R_3$ représente un atome d'hydrogène, un radical alkyle inférieur en $C_1$ à $C_4$, un radical carbonyle lié à un autre radical $R_9$

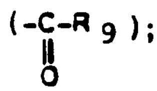

$R_4$ représente un atome d'hydrogène, un radical alkyle intérieur en $C_1$ à $C_4$ ;

$R_5$ représente un atome d'hydrogène, un radical méthyle, un radical hydroxy ;

$R_6$ représente un atome d'hydrogène, un radical méthyle, un radical nitro ($NO_2$) ;

$R_7$ représente un atome d'hydrogène, un radical alkyle inférieur en $C_1$ à $C_4$ ;

$R_8$ représente un atome d'hydrogène, un radical méthyle;

$R_9$ représente un radical alkoxy ;

à l'exception toutefois du composé de formule I, dans laquelle:

X représente CH,

Y représente N,

Z représente S, et

$R_1$ à $R_7$ représentent H,

caractérisé par la mise en oeuvre d'au moins l'une des opérations suivantes :

A) Par réaction d'un composé de formule générale II

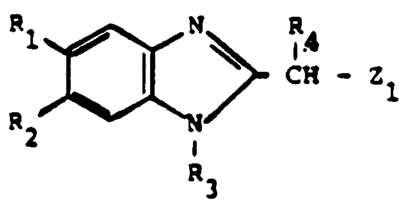

II

avec un composé de formule générale III

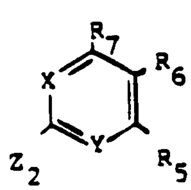

III

dans lesquelles les radicaux X, Y et $R_1$ à $R_7$ ont les significations données précédemment, et l'un des deux radicaux $Z_1$ et $Z_2$ est constitué par le radical -SH et l'autre est un groupe partant notamment choisi parmi les halogènes, de préférence le fluor, le chlore ou le brome ; des radicaux formés par des groupes estérifiés et qui soient réactifs notamment acétyloxy, tosyloxy ou mésyloxy ; ou encore des groupes alkylthio ou alkylsulfinyle, par exemple méthylthio ou méthylsulfinyle ; ou bien

B) Par réaction d'un composé de formule générale II avec un composé de formule générale III, dans lesquelles : X et $R_1$ à $R_7$ ont les significations mentionnées précédemment,

Y représente un groupe N-oxyde (N → O)

$Z_1$ est constitué par le radical -SH et $Z_2$ représente un atome d'hydrogène, on prépare un composé de formule générale I dans laquelle X et $R_1$ à $R_7$ ont les significations données précédemment,

Y représente un atome d'azote et Z représente un atome de soufre ; ou bien

C) Par réaction d'un composé de formule générale IV

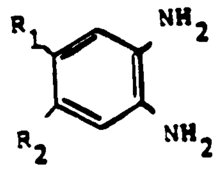

IV

avec un composé de formule générale V

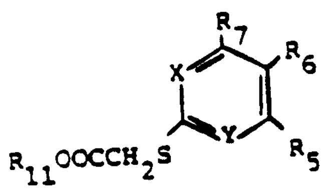

V

dans lesquelles les radicaux X, Y, $R_1$, $R_2$, $R_5$, $R_6$ et $R_7$ ont les significations données précédemment, et $R_{11}$ représente un atome d'hydrogène ou un radical alkyle inférieur, tel que méthyle ou éthyle,

on prépare un composé de formule générale I dans laquelle X, Y, $R_1$, $R_2$, $R_5$, $R_6$ et $R_7$ ont les significations données précédemment, $R_3$ et $R_4$ représentent un atome d'hydrogène et Z représente un atome de soufre, ou bien

D) Par oxydation, avec une quantité stoechiométrique d'un agent oxydant, des composés de formule générale I dans laquelle X, Y et $R_1$ à $R_7$ ont les significations données précédemment, et Z représente un atome de soufre, on prépare des composés de formule générale I dans laquelle X, Y et $R_1$ à $R_7$ ont les significations données précédemment, et Z représente un groupe sulfinyle (S → O);

Ledit agent oxydant étant de préférence choisi parmi le peroxyde d'hydrogène, les peracides tels que l'acide m-chloroperbenzoïque, l'acide nitrique, le métapériodate de sodium, l'acide chromique, le dioxyde de manganèse, le chlore, le brome ou le chlorure de sulfuryle ; ou bien

E) Par oxydation, avec une quantité d'un agent oxydant deux à trois fois supérieure à la quantité stoechiométrique des composés de formule générale I dans laquelle X, Y et $R_1$ à $R_7$ ont les significations données précédemment, et Z représente un atome de soufre,

on prépare des composés de formule générale I dans laquelle X, Y et $R_1$ à $R_7$ ont les significations données précédemment, et Z représente un groupe sulfonyle (O ← S → O).

Ledit agent oxydant étant de préférence choisi parmi le peroxyde d'hydrogène, les peracides tels que l'acide m-chloroperbenzoïque, l'acide nitrique, le métapériodate de sodium, l'acide chromique, le dioxyde de manganèse, le chlore, le brome ou le chlorure de sulfuryle ;

Les produits de formule générale I obtenus par les procédés précédents pouvant, le cas échéant, être transformés ultérieurement.

**2.** Procédé selon la revendication 1 caractérisé en ce que les transformations ultérieures sont effectuées par la mise en oeuvre d'au moins l'une des opérations suivantes :

F) Par nitration, dans un mélange d'acide sulfurique et nitrique, des composés de formule générale I dans laquelle $R_2$ et $R_3$ représentent des atomes d'hydrogène et X, Y, Z, $R_1$, $R_4$ à $R_7$ ont les significations données précédemment, on prépare des composés de formule générale I dans laquelle X, Y, Z, $R_1$ et $R_4$ à $R_7$ ont les significations données précédemment, $R_2$ représente un groupe nitro($NO_2$) et $R_3$ représente un atome d'hydrogène, ou bien

G) Pour la préparation des composés de formule générale I, où $R_1$ ou $R_2$ est méthoxycarbonyle

($COOCH_3$), $R_3$ représente un atome d'hydrogène et $R_2$ ou $R_1$, X, Y, Z et $R_4$ à $R_7$ ont les significatins données précédemment,
on fait réagir un composé de formule générale I dans laquelle $R_1$ ou $R_2$ représente un radical carboxyle (COOH), $R_3$ représente un atome d'hydrogène, et $R_2$ ou $R_1$, X, Y, Z et $R_4$ à $R_7$ ont les significations données précédemment, avec du méthanol, ou bien
H) Pour la préparation des composés de formule générale I dans laquelle $R_3$ représente un radical alkyle inférieur en $C_1$ à $C_4$ et X, Y, Z, $R_1$, $R_2$ et $R_4$ à $R_7$ ont les significations données précédemment,
on fait réagir un composé de formule générale I dans laquelle $R_3$ représente un atome d'hydrogène et X, Y, Z, $R_1$, $R_2$ et $R_4$ à $R_7$ ont les significations données précédemment,
avec un agent alkylant, tel que sulfate de diméthyle, diméthylformamide diméthylacétal ou un halogénure d'alkyle dans lequel les halogènes préférés sont le chlore, le brome et l'iode, ou bien
I) Pour la préparation des composés de formule générale I dans laquelle $R_3$ représente un radical carbonyle lié à un autre radical $R_9$

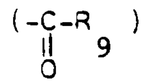

et X, Y, Z, $R_1$, $R_2$ et $R_4$ à $R_9$ ont les significations données précédemment,
on fait réagir un composé de formule générale I dans laquelle $R_3$ représente un atome d'hydrogène et X, Y, Z, $R_1$, $R_2$ et $R_4$ à $R_7$ ont les significations données précédemment, avec un composé de formule générale VI

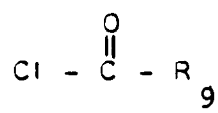

VI

dans laquelle $R_9$ a la signification donnée précédemment.

## Claims

**Claims for the following Contracting States : BE, CH, DE, FR, GB, IT LI, LU, NL, SE**

**1.** Derivatives of 2-alkylbenzimidazole corresponding to general formula I

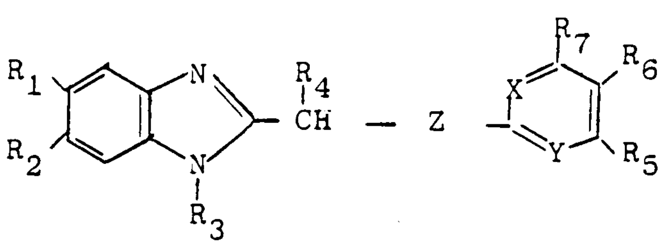

I

as well as the therapeutically acceptable salts thereof, in which

| | |
|---|---|
| X | represents a nitrogen atom (N) or a carbon atom bound to a further radical $R_8$ ($C-R_8$); |
| Y | represents a nitrogen atom (N) or an N - oxide group ($N \rightarrow O$); |
| Z | represents a sulphur atom (S), a sulphinyl group ($S \rightarrow O$) or a sulphonyl group ($O \leftarrow S \rightarrow O$); |
| $R_1$ and $R_2$, | which may be the same or different, represent a hydrogen atom, a halogen, a linear or branched $C_1$ to $C_4$ lower alkyl radical, a nitro group ($NO_2$), a trifluoromethyl group ($CF_3$), a $C_1$ to $C_4$ alkoxy a methylcarbonyl radical, a methoxycarbonyl radical, a benzoyl radical; |
| $R_3$ | represents a hydrogen atom, a $C_1$ to $C_4$ lower alkyl radical, a carbonyl radical bound to a further radical $R_9$ |

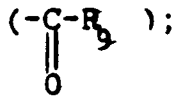

| | |
|---|---|
| $R_4$ | represents a hydrogen atom, a $C_1$ to $C_4$ lower alkyl radical; |
| $R_5$ | represents a hydrogen atom, a methyl radical, a hydroxy radical, |
| $R_6$ | represents a hydrogen atom, a methyl radical, a nitro-radical ($NO_2$), |
| $R_7$ | represents a hydrogen atom, a $C_1$ to $C_4$ lower alkyl radical, |
| $R_8$ | represents a hydrogen atom, a methyl radical; |
| $R_9$ | represents an alkoxy radical, |
| $R_{10}$ | |

but with the exception of the compound corresponding to formula I, in which

| | |
|---|---|
| X | represents CH, |
| Y | represents N, |
| Z | represents S, and |
| $R_1$ to $R_7$ | represent H. |

**2.** The compounds corresponding to general formula I, according to claim 1, selected from the following group:

-2-(2-benzimidazolylmethylthio)-pyrimidine
-2-(2-benzimidazolylmethylthio)-4-hydroxy-6-(n-propyl-pyrimidine
2-(2-benzimidazolylmethylthio)-4,6-dimethylpyrimidine
-2-(5-nitro-2-pyridylthiomethyl)-benzimidazole
-5-methyl-2-(2-pyridylthiomethyl)-benzimidazole
-5,6-dimethyl-2-(2-pyridylthiomethyl)-benzimidazole
-2-(1-oxy-2-pyridylthiomethyl)-benzimidazole
-5-chloro-2-(2-pyridylthiomethyl)-benzimidazole
-5-trifluoromethyl-2-(2-pyridylthiomethyl)-benzimidazole
-5-benzoyl-2-(2-pyridylthiomethyl)-benzimidazole
-5-methoxy-2-(2-pyridylthiomethyl)-benzimidazole
-2-[2-pyridylthio-(methyl)-methyl]-benzimidazole
-5-benzoyl-2-(3,5-dimethyl-2-pyridylthiomethyl)-benzimidazole
-5-benzoyl-2-(3-methyl-2-pyridylthiomethyl)-benzimidazole
-2-(3-methyl-2-pyridylthiomethyl)-benzimidazole
-2-(4-methyl-2-pyridylthiomethyl)-benzimidazole
-5-trifluoromethyl-2-(4-methyl-2-pyridylthiomethyl)
-benzimidazole
-5-methoxycarbonyl-2-(4-methyl-2-pyridylthiomethyl)
-benzimidazole
-5-methoxycarbonyl-2-(2-pyridylthiomethyl)-benzimidazole
-5-benzoyl-2-(4-methyl-2-pyridylthiomethyl)-benzimidazole
-5-methoxy-2-(4-methyl-2-pyridylthiomethyl)-benzimidazole
-5-acetyl-2-(4-methyl-2-pyridylthiomethyl)-benzimidazole
-5-methyl-2-(4-methyl-2-pyridylthiomethyl)-benzimidazole
-5,6-dimethyl-2-(4-methyl-2-pyridylthiomethyl)-benzimidazole
-5-chloro-2-(4-methyl-2-pyridylthiomethyl)-benzimidazole

**3.** The compounds corresponding to general formula I, according to claim 1, selected from the following group:

-5-benzoyl-2-(2-pyridylsulphinylmethyl)-benzimidazole
-2-(2-pyridylsulphinylmethyl)-benzimidazole
-2-(5-nitro-2-pyridylsulphinylmethyl)-benzimidazole
-5-methyl-2-(pyridylsulphinylmethyl)-benzimidazole
-5,6-dimethyl-2-(2--pyridylsulphinylmethyl)-benzimidazole
-5-chloro-2-(2-pyridylsulphinylmethyl)-benzimidazole

-2-(2-benzimidazolylmethylsulphinyl)-4,6-dimethylpyrimidine
-5-trifluoromethyl-2-(2-pyridylsulphinylmethyl)-benzimidazole
-5-methoxy-2-(2-pyridylsulphinylmethyl)-benzimidazole
-erythro-2-[2-pyridylsulphinyl-(methyl)-methyl]-benzimidazole
-threo-2-[2-pyridylsulphinyl-(methyl)-methyl]-benzimidazole
-2-(1-oxy-2-pyridylsulphinylmethyl)-benzimidazole
-2-(2-benzimidazolylmethylsulphinyl)-pyrimidine
-5-benzoyl -2-(3,5-dimethyl-2-pyridylsulphinylmethyl)-benzimidazole
-5-benzoyl-2-(3-methyl-2-pyridylsulphinylmethyl)-benzimidazole
-2-(3-methyl-2-pyridylsulphinylmethyl)-benzimidazole
-5-benzoyl-2-(4-methyl-2- pyridylsulphinylmethyl)-benzimidazole
-5-methoxycarbonyl-2-(2-pyridylsulphinylmethyl)-benzimidazole
-2-(4-methyl-2-pyridylsulphinylmethyl)-benzimidazole
-5-trifluoromethyl-2-(4-methyl-2-pyridylsulphinylmethyl)-benzimidazole
-5-methoxycarbonyl-2-(4-methyl-2-pyridylsulphinymethyl)-benzimidazole
-5-methoxy-2-(4-methyl-2-pyridylsulphinylmethyl)-benzimidazole
-5-acetyl-2-(4-methyl-2-pyridylsulphinylmethyl)-benzimidazole
-5-methyl-2-(4-methyl-2-pyridylsulphinylmethyl)-benzimidazole
-5,6-dimethyl-2-(4-methyl-2-pyridylsulphinylmethyl)-benzimidazole
-5-chloro-2-(4-methyl-2-pyridylsulphinylmethyl)-benzimidazole

4. The compounds corresponding to general formula I, according to claim 1, selected from the following group:

-5-chloro-2-(2-pyridylsulphonylmethyl)-benzimidazole
-2-(2-pyridylsulphonylmethyl)-benzimidazole
-5-methyl-2-(2-pyridylsulphonylmethyl)-benzimidazole
-5,6-dimethyl-2-(2-pyridylsulphonylmethyl)-benzimidazole
-2-[2-pyridylsulphonyl-(methyl)-methyl]-benzimidazole

5. The compounds corresponding to general formula I, according to claim 1, selected from the following group:

-5-nitro-2-(2-pyridylsulphinylmethyl)benzimidazole
-5-benzoyl-1-methyl-2-(2-pyridylsulphinylmethyl)-benzimidazole
-6-benzoyl-1-methyl-2-(2-pyridylsulphinylmethyl)-benzimidazole
-5-benzoyl-1-ethoxycarbonyl-2-(2-pyridylsulphinylmethyl)-benzimidazole
-6-benzoyl-1-ethoxycarbonyl-2-(2-pyridylsulphinylmethyl)-benzimidazole
-1-ethyl-2-(2-pyridylsulphinylmethyl)-benzimidazole.

6. A process for the preparation of the compounds according to one of claims 1 to 5 , characterised by carrying out at least one of the following processes:

A) by reaction of a compound corresponding to general formula II

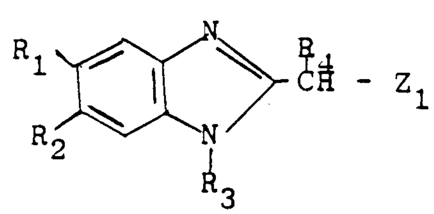

II

with a compound corresponding to general formula III

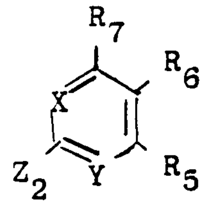

III

in which the radicals X,Y and $R_1$ to $R_7$ have the meanings given in claim 1, and one of the two radicals $Z_1$ and $Z_2$ is constituted by the radical -SH and the other is a leaving group selected, in particular, from among the halogens, preferably fluorine, chlorine or bromine; radicals formed by esterified groups which are reactive, in particular acetyloxy, tosyloxy or mesyloxy; or furthermore alkylthio or alkylsulphinyl groups, for example methylthio or methylsulphinyl; or

B) by reaction of a compound corresponding to general formula II with a compound corresponding to general formula III in which: X and $R_1$ to $R_7$ have the meanings given in claim 1, Y represents an N-oxide group (N → O),$Z_1$ is constituted by the radical -SH and $Z_2$ represents a hydrogen atom,

a compound corresponding to general formula I in which X and $R_1$ to $R_7$ have the meanings given in claim 1, Y represents a nitrogen atom and Z represents a sulphur atom is prepared; or

C) by reaction of a compound corresponding to general formula IV

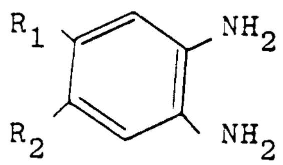

IV

with a compound corresponding to general formula V

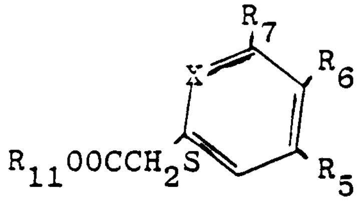

V

in which the radicals X, Y, $R_1$, $R_2$, $R_5$, $R_6$ and $R_7$ have the meanings given in claim 1 and $R_{11}$ represents a hydrogen atom or a lower alkyl radical such as methyl or ethyl,

a compound corresponding to general formula I in which X, Y, $R_1$, $R_2$, $R_5$, $R_6$, and $R_7$ have the meanings given in claim 1, $R_3$ and $R_4$ represent a hydrogen atom and Z represents a sulphur atom is prepared; or

D) by oxidation with a stoichiometric quantity of an oxidizing agent of the compounds corresponding to general formula I in which X, Y and $R_1$ to $R_7$ have the meanings given in claim 1 and Z represents a sulphur atom, compounds corresponding to general formula 1 in which X, Y and $R_1$ to $R_7$ have the meanings given in claim 1 and Z represents a sulphinyl group (S → O) are prepared;

said oxidizing agent preferably being selected from hydrogen peroxide, peracids much as m-chloroperbenzoic acid, nitric acid, sodium metaperiodate, chromic acid, manganese dioxide, chlorine, bromine or sulphuryl chloride; or

E) by oxidation with a quantity of an oxidizing agent two to three times greater than the stoichiometric quantity of the compounds corresponding to general formula I in which X, Y and $R_1$ to $R_7$ have the meanings given in claim 1 and Z represents a sulphur atom,

the compounds corresponding to general formula 1 in which X, Y and $R_1$ to $R_7$ have the meanings

given in claim 1 and Z represents a sulphonyl group (O ← S → O) are prepared; said oxidizing agent preferably being selected from hydrogen peroxide, peracids such as m-chloroperbenzoic acid, nitric acid, sodium metaperiodate, chromic acid, manganese dioxide, chlorine, bromine or sulphuryl chloride.

**7.** A process for the preparation of the compounds according to one of claims 1 to 5, characterised by carrying out at least one of the following processes:

F) by nitration in a mixture of sulphuric and nitric acid of the compounds corresponding to general formula I in which $R_2$ and $R_3$ represent hydrogen atoms and X,Y, Z, $R_1$, $R_4$ to $R_7$ have the meanings given in claim 1, the compounds corresponding to general formula I in which X, Y, Z, $R_1$ and $R_4$ to $R_7$ have the meanings given in claim 1, $R_2$ represents a nitro group ($NO_2$) and $R_3$ represents a hydrogen atom are prepared, or

G) for the preparation of the compounds corresponding to general formula I wherein $R_1$ or $R_2$ is methoxycarbonyl ($COOCH_3$), $R_3$ represents a hydrogen atom and $R_2$ or $R_1$, X, Y, Z and $R_4$ to $R_7$ have the meanings given in claim 1, a compound corresponding to general formula I in which $R_1$ or $R_2$ represents a carboxyl radical (COOH), $R_3$ represents a hydrogen atom and $R_2$ or $R_1$, X, Y, Z, and $R_4$ to $R_7$ have the meanings given in claim 1 is reacted with methanol or

H) for the preparation of the compounds corresponding to general formula I in which $R_3$ represents a $C_1$ to $C_4$ lower alkyl radical and X, Y, Z, $R_1$, $R_2$ and $R_4$ to $R_7$ have the meanings given in claim 1, a compound corresponding to general formula I in which $R_3$ represents a hydrogen atom and X, Y, Z, $R_1$, and $R_3$ to $R_7$ have the meanings given in claim 1, is reacted with an alkylating agent such as dimethyl sulphate, dimethyl formamide, dimethyl acetal or an alkyl halide in which the preferrred halogens are chlorine, bromine and iodine, or

I) for the preparation of the compounds corresponding to general formula I in which $R_3$ represents a carbonyl radical bound to a different radical $R_9$

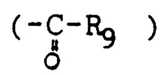

and X, Y, Z, $R_1$, $R_2$ and $R_4$ to $R_{10}$ have the meanings given in claim 1, a compound corresponding to general formula I in which $R_3$ represents a hydrogen atom and X, Y, Z, $R_1$, $R_2$ and $R_4$ to $R_7$ have the meanings given in claim 1 is reacted with a compound corresponding to general formula VI

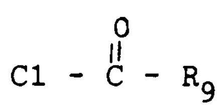

VI

in which $R_9$ has the meaning given in claim 1.

**8.** As medicaments derivatives corresponding to general formula I and the therapeutically acceptable salts thereof according to one of claims 1 to 5 , in particular as medicaments intended for the treatment of gastro-intestinal illnesses, principally indicated as gastric acid secretion inhibitors and as cytoprotective agents.

**9.** Pharmaceutical compositions, characterised in that they contain, in addition to a pharmaceutically acceptable carrier, at least one derivative corresponding to general formula I or one of the physiologically acceptable salts thereof, according to one of claims 1 to 5.

**10.** Use of the derivatives corresponding to general formula I and of their physiologically acceptable salts according to one of claims 1 to 5 for the production of medicaments intended for the treatment of gastro-intestinal illnesses, in particular for the production of gastric acid secretion inhibitors and cytoprotective agents.

**Claims for the following Contracting States : AT, GR**

**1.** A process for the preparation of derivatives of 2-alkylbenzimidazole corresponding to general formula I

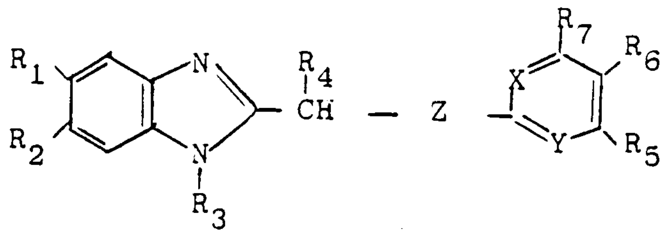

I

as well as the therapeutically acceptable salts thereof, in which

| | |
|---|---|
| X | represents a nitrogen atom (N) or a carbon atom bound to a further radical $R_8$ ($C-R_8$); |
| Y | represents a nitrogen atom (N) or an N - oxide group ($N \rightarrow O$); |
| Z | represents a sulphur atom (S), a sulphinyl group ($S \rightarrow O$) or a sulphonyl group ($O \leftarrow S \rightarrow O$); |
| $R_1$ and $R_2$, | which may be the same or different, represent a hydrogen atom, a halogen, a linear or branched $C_1$ to $C_4$ lower alkyl radical, a nitro group ($NO_2$), a trifluoromethyl group ($CF_3$), a $C_1$ to $C_4$ alkoxy a methylcarbonyl radical, a methoxycarbonyl radical, a benzoyl radical; |
| $R_3$ | represents a hydrogen atom, a $C_1$ to $C_4$ lower alkyl radical, a carbonyl radical bound to a further radical $R_9$ |

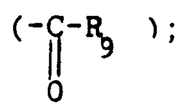

| | |
|---|---|
| $R_4$ | represents a hydrogen atom, a $C_1$ to $C_4$ lower alkyl radical; |
| $R_5$ | represents a hydrogen atom, a methyl radical, a hydroxy radical, |
| $R_6$ | represents a hydrogen atom, a methyl radical, a nitro-radical ($NO_2$), |
| $R_7$ | represents a hydrogen atom, a $C_1$ to $C_4$ lower alkyl radical, |
| $R_8$ | represents a hydrogen atom, a methyl radical; |
| $R_9$ | represents an alkoxy radical, |

but with the exception of the compound corresponding to formula I, in which

| | |
|---|---|
| X | represents CH, |
| Y | represents N, |
| Z | represents S, and |
| $R_1$ to $R_7$ | represent H, |

characterised by carrying out at least one of the following processes:

A) by reaction of a compound corresponding to general formula II

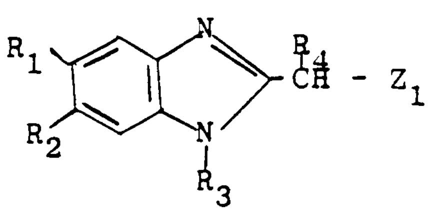

II

with a compound corresponding to general formula III

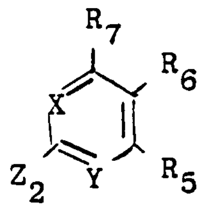

III

in which the radicals X,Y and $R_1$ to $R_7$ have the meanings given in claim 1, and one of the two radicals $Z_1$ and $Z_2$ is constituted by the radical -SH and the other is a leaving group selected, in particular, from among the halogens, preferably fluorine, chlorine or bromine; radicals formed by esterified groups which are reactive, in particular acetyloxy, tosyloxy or mesyloxy; or furthermore alkylthio or alkylsulphinyl groups, for example methylthio or methylsulphinyl; or

B) by reaction of a compound corresponding to general formula II with a compound corresponding to general formula III in which: X and $R_1$ to $R_7$ have the meanings given previously , Y represents an N-oxide group (N → O),$Z_1$ is constituted by the radical -SH and $Z_2$ represents a hydrogen atom,

a compound corresponding to general formula I in which X and $R_1$ to $R_7$ have the meanings given previously , Y represents a nitrogen atom and Z represents a sulphur atom is prepared; or

C) by reaction of a compound corresponding to general formula IV

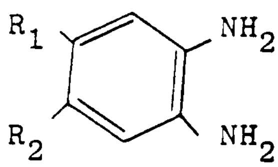

IV

with a compound corresponding to general formula V

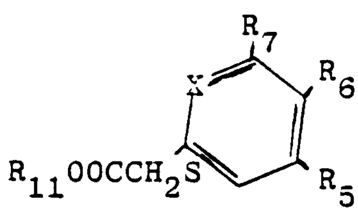

V

in which the radicals X, Y, $R_1$, $R_2$, $R_5$, $R_6$ and $R_7$ have the meanings given previously and $R_{11}$ represents a hydrogen atom or a lower alkyl radical such as methyl or ethyl,

a compound corresponding to general formula I in which X, Y, $R_1$, $R_2$, $R_5$, $R_6$, and $R_7$ have the meanings given previously , $R_3$ and $R_4$ represent a hydrogen atom and Z represents a sulphur atom is prepared; or

D) by oxidation with a stoichiometric quantity of an oxidizing agent of the compounds corresponding to general formula I in which X, Y and $R_1$ to $R_7$ have the meanings given in claim 1 and Z represents a sulphur atom, compounds corresponding to general formula 1 in which X, Y and $R_1$ to $R_7$ have the meanings given previously and Z represents a sulphinyl group (S → O) are prepared

said oxidizing agent preferably being selected from hydrogen peroxide, peracids much as m-chloroperbenzoic acid, nitric acid, sodium metaperiodate, chromic acid, manganese dioxide, chlorine, bromine or sulphuryl chloride; or

E) by oxidation with a quantity of an oxidizing agent two to three times greater than the stoichiometric quantity of the compounds corresponding to general formula I in which X, Y and $R_1$ to $R_7$ have the meanings given previously and Z represents a sulphur atom,

the compounds corresponding to general formula 1 in which X, Y and $R_1$ to $R_7$ have the meanings given previously and Z represents a sulphonyl group (O ← S → O) are prepared;

said oxidizing agent preferably being selected from hydrogen peroxide, peracids such as m-chloroperbenzoic acid, nitric acid, sodium metaperiodate, chromic acid, manganese dioxide, chlorine, bromine or sulphuryl chloride; or
The compounds of general formula I obtained by the previous processes being, if need be, transformed subsequently.

**2.** Process according to claim 1, characterized by carrying at least one of the following processes:
F) by nitration in a mixture of sulphuric and nitric acid of the compounds corresponding to general formula I in which $R_2$ and $R_3$ represent hydrogen atoms and X,Y, Z, $R_1$, $R_4$ to $R_7$ have the meanings given previously , the compounds corresponding to general formula I in which X, Y, Z, $R_1$ and $R_4$ to $R_7$ have the meanings given previously , $R_2$ represents a nitro group ($NO_2$) and $R_3$ represents a hydrogen atom are prepared, or
G) for the preparation of the compounds corresponding to general formula I wherein $R_1$ or $R_2$ is methoxycarbonyl ($COOCH_3$) $R_3$ represents a hydrogen atom and $R_2$ or $R_1$, X, Y, Z and $R_4$ to $R_7$ have the meanings given in claim 1, a compound corresponding to general formula I in which $R_1$ or $R_2$ represents a carboxyl radical (COOH), $R_3$ represents a hydrogen atom and $R_2$ or $R_1$, X, Y, Z, and $R_4$ to $R_7$ have the meanings given previously is reacted with methanol or
H) for the preparation of the compounds corresponding to general formula I in which $R_3$ represents a $C_1$ to $C_4$ lower alkyl radical and X, Y, Z, $R_1$, $R_2$ and $R_4$ to $R_7$ have the meanings given in claim 1, a compound corresponding to general formula I in which $R_3$ represents a hydrogen atom and X, Y, Z, $R_1$, and $R_3$ to $R_7$ have the meanings given previously is reacted with an alkylating agent such as dimethyl sulphate, dimethyl formamide, dimethyl acetal or an alkyl halide in which the preferrred halogens are chlorine, bromine and iodine, or
I) for the preparation of the compounds corresponding to general formula I in which $R_3$ represents a carbonyl radical bound to a different radical $R_9$

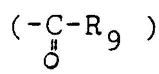

and X, Y, Z, $R_1$, $R_2$ and $R_4$ to $R_{10}$ have the meanings given previously , a compound corresponding to general formula I in which $R_3$ represents a hydrogen atom and X, Y, Z, $R_1$, $R_2$ and $R_4$ to $R_7$ have the meanings given previously is reacted with a compound corresponding to general formula VI

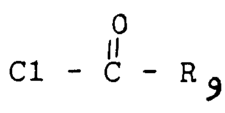

VI

in which $R_{10}$ has the meaning given previously.

**Patentansprüche**
**Patentansprüche für folgende Vertragsstaaten : BE, CH, DE, FR, GB, IT, LI, LU, NL, SE**

**1.** 2-Alkylbenzimidazol-Derivate der allgemeinen Formel I

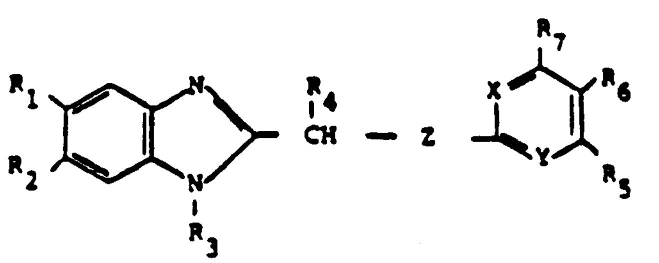

I

sowie ihre therapeutisch akzeptablen Salze, in der

X ein Stickstoffatom (N) oder ein an ein anderes Radikal $R_8$ ($C-R_8$) gebundenes Kohlenstoffatom darstellt;

| | |
|---|---|
| Y | ein Stickstoffatom (N) oder eine N-Oxid-Gruppe (N→O) bedeutet; |
| Z | ein Schwefelatom (S), eine Sulfinylgruppe (S→O) oder eine Sulfonylgruppe (O←S→O) ist; |
| $R_1$ und $R_2$, | gleich oder verschieden, ein Wasserstoffatom, ein Halogenatom, ein lineares oder verzweigtes, niederes Alkyl-Radikal mit 1 bis 4 Kohlenstoffatomen, eine Nitrogruppe ($NO_2$), eine Trifluormethylgruppe ($CF_3$), ein niederes Alkoxy-Radikal mit 1 bis 4 Kohlenstoffatomen, ein Methylcarbonyl-Radikal, ein Methoxycarbonyl-Radikal oder ein Benzoyl-Radikal darstellen; |
| $R_3$ | ein Wasserstoffatom, ein niederes Alkyl-Radikal mit 1 bis 4 Kohlenstoffatomen, ein an ein anderes Radikal $R_9$ (-CO-$R_9$) gebundenes Carbonyl-Radikal bedeutet; |
| $R_4$ | ein Wasserstoffatom oder ein niederes Alkyl-Radikal mit 1 bis 4 Kohlenstoffatomen ist; |
| $R_5$ | ein Wasserstoffatom, ein Methyl-Radikal oder ein Hydroxy-Radikal darstellt; |
| $R_6$ | ein Wasserstoffatom, ein Methyl-Radikal oder ein Nitro-Radikal ($NO_2$) bedeutet; |
| $R_7$ | ein Wasserstoffatom oder ein niederes Alkyl-Radikal mit 1 bis 4 Kohlenstoffatomen ist; |
| $R_8$ | ein Wasserstoffatom oder ein Methyl-Radikal darstellt; und |
| $R_9$ | ein Alkoxy-Radikal bedeutet, |

jedoch mit der Ausnahme der Verbindung der Formel I, in der

| | |
|---|---|
| X | CH darstellt, |
| Y | N bedeutet, |
| Z | Schwefel ist, und |
| $R_1$ bis $R_7$ | Wasserstoff darstellen. |

**2.** Verbindungen der allgemeinen Formel I nach Anspruch 1, ausgewählt aus der folgenden Gruppe:
- 2-(2-Benzimidazolylmethylthio)-pyrimidin,
- 2-(2-Benzimidazolylmethylthio)-4-hydroxy-6-(n-propyl)-pyrimidin,
- 2-(2-Benzimidazolylmethylthio)-4,6-dimethyl-pyrimidin,
- 2-(5-Nitro-2-pyridylthiomethyl)-benzimidazol,
- 5-Methyl-2-(2-pyridylthiomethyl)-benzimidazol,
- 5,6-Dimethyl-2-(2-pyridylthiomethyl)-benzimidazol,
- 2-(1-Oxid-2-pyridylthiomethyl)-benzimidazol,
- 5-Chlor-2-(2-pyridylthiomethyl)-benzimidazol,
- 5-Trifluormethyl-2-(2-pyridylthiomethyl)-benzimidazol,
- 5-Benzoyl-2-(2-pyridylthiomethyl)-benzimidazol,
- 5-Methoxy-2-(2-pyridylthiomethyl)-benzimidazol,
- 2-[2-Pyridylthio-(methyl)-methyl]-benzimidazol,
- 5-Benzoyl-2-(3,5-dimethyl-2-pyridylthiomethyl)-benzimidazol,
- 5-Benzoyl-2-(3-methyl-2-pyridylthiomethyl)-benzimidazol,
- 2-(3-Methyl-2-pyridylthiomethyl)-benzimidazol,
- 2-(4-Methyl-2-pyridylthiomethyl)-benzimidazol,
- 5-Trifluormethyl-2-(4-methyl-2-pyridylthiomethyl)-benzimidazol,
- 5-Methoxycarbonyl-2-(4-methyl-2-pyridylthiomethyl)-benzimidazol,
- 5-Methoxycarbonyl-2-(2-pyridylthiomethyl)-benzimidazol,
- 5-Benzoyl-2-(4-methyl-2-pyridylthiomethyl)-benzimidazol,
- 5-Methoxy-2-(4-methyl-2-pyridylthiomethyl)-benzimidazol,
- 5-Acetyl-2-(4-methyl-2-pyridylthiomethyl)-benzimidazol,
- 5-Methyl-2-(4-methyl-2-pyridylthiomethyl)-benzimidazol,
- 5,6-Dimethyl-2-(4-methyl-2-pyridylthiomethyl)-benzimidazol,
- 5-Chlor-2-(4-methyl-2-pyridylthiomethyl)-benzimidazol.

**3.** Verbindungen der allgemeinen Formel I nach Anspruch 1, ausgewählt aus der folgenden Gruppe:
- 5-Benzoyl-2-(2-pyridylsulfinylmethyl)-benzimidazol,
- 2-(2-Pyridylsulfinylmethyl)-benzimidazol,
- 2-(5-Nitro-2-pyridylsulfinylmethyl)-benzimidazol,
- 5-Methyl-2-(2-pyridylsulfinylmethyl)-benzimidazol,
- 5,6-Dimethyl-2-(2-pyridylsulfinylmethyl)-benzimidazol,
- 5-Chlor-2-(2-pyridylsulfinylmethyl)-benzimidazol,

- 2-(2-Benzimidazolylmethylsulfinyl)-4,6-dimethylpyrimidin,
- 5-Trifluormethyl-2-(2-pyridylsulfinylmethyl)-benzimidazol,
- 5-Methoxy-2-(2-pyridylsulfinylmethyl)-benzimidazol,
- erythro-2-[2-Pyridylsulfinyl-(methyl)-methyl]-benzimidazol,
- threo-2-[2-Pyridylsulfinyl-(methyl)-methyl]-benzimidazol,
- 2-(1-Oxid-2-pyridylsulfinylmethyl)-benzimidazol,
- 2-(2-Benzimidazolylmethylsulfinyl)-pyrimidin,
- 5-Benzoyl-2-(3,5-dimethyl-2-pyridylsulfinylmethyl)-benzimidazol,
- 5-Benzoyl-2-(3-methyl-2-pyridylsulfinylmethyl)-benzimidazol,
- 2-(3-Methyl-2-pyridylsulfinylmethyl)-benzimidazol,
- 5-Benzoyl-2-(4-methyl-2-pyridylsulfinylmethyl)-benzimidazol,
- 5-Methoxycarbonyl-2-(2-pyridylsulfinylmethyl)-benzimidazol,
- 2-(4-Methoxy-2-pyridylsulfinylmethyl)-benzimidazol,
- 5-Trifluormethyl-2-(4-methyl-2-pyridylsulfinylmethyl)-benzimidazol,
- 5-Methoxycarbonyl-2-(4-methyl-2-pyridylsulfinylmethyl)-benzimidazol,
- 5-Methoxy-2-(4-methyl-2-pyridylsulfinylmethyl)-benzimidazol,
- 5-Acetyl-2-(4-methyl-2-pyridylsulfinylmethyl)-benzimidazol,
- 5-Methyl-2-(4-methyl-2-pyridylsulfinylmethyl)-benzimidazol,
- 5,6-Dimethyl-2-(4-methyl-2-pyridylsulfinylmethyl)-benzimidazol,
- 5-Chlor-2-(4-methyl-2-pyridylsulfinylmethyl)-benzimidazol.

**4.** Verbindungen der allgemeinen Formel I nach Anspruch 1, ausgewählt aus der folgenden Gruppe:
- 5-Chlor-2-(2-pyridylsulfonylmethyl)-benzimidazol,
- 2-(2-Pyridylsulfonylmethyl)-benzimidazol,
- 5-Methyl-2-(2-pyridylsulfonylmethyl)-benzimidazol,
- 5,6-Dimethyl-2-(2-pyridylsulfonylmethyl)-benzimidazol,
- 2-[2-Pyridylsulfonyl-(methyl)-methyl]-benzimidazol.

**5.** Verbindungen der allgemeinen Formel I nach Anspruch 1, ausgewählt aus der folgenden Gruppe:
- 5-Nitro-2-(2-pyridylsulfinylmethyl)-benzimidazol
- 5-Benzoyl-1-methyl-2-(2-pyridylsulfinylmethyl)-benzimidazol,
- 6-Benzoyl-1-methyl-2-(2-pyridylsulfinylmethyl)-benzimidazol,
- 5-Benzoyl-1-ethoxycarbonyl-2-(2-pyridylsulfinylmethyl)-benzimidazol,
- 6-Benzoyl-1-ethoxycarbonyl-2-(2-pyridylsulfinylmethyl)-benzimidazol,
- 1-Ethyl-2-(2-pyridylsulfinylmethyl)-benzimidazol.

**6.** Verfahren zur Herstellung der Verbindungen nach einem der Ansprüche 1 bis 5, dadurch gekennzeichnet, daß mindestens eine der nachfolgenden Operationen durchgeführt wird:

A) Durch Reaktion einer Verbindung der allgemeinen Formel II

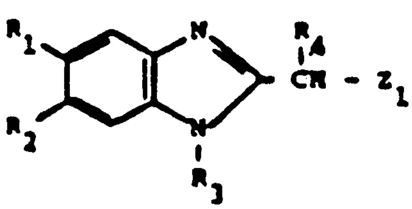

II

mit einer Verbindung der allgemeinen Formel III

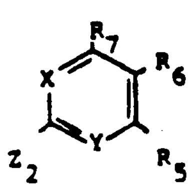

III

in denen die Radikale X, Y und $R_1$ bis $R_7$ die in Anspruch 1 angegebenen Bedeutungen besitzen und eines der zwei Radikale $Z_1$ und $Z_2$ durch das Radikal SH und das andere durch eine Abgangsgruppe gebildet wird, insbesondere ausgewählt unter den Halogenen, vorzugsweise Fluor, Chlor und Brom; durch aus veresterten Gruppen gebildeten reaktiven Radikalen, insbesondere Acetyloxy, Tosyloxy oder Mesyloxy, oder auch durch Alkylthio- oder Alkylsulfinyl-Gruppen, beispielsweise Methylthio oder Methylsulfinyl; oder

B) durch Reaktion einer Verbindung der allgemeinen Formel II mit einer Verbindung der allgemeinen Formel III, in denen X und $R_1$ bis $R_7$ die in Anspruch 1 angegebenen Bedeutungen besitzen, Y eine N-Oxid-Gruppe (N→O) darstell, $Z_1$ durch das Radikal -SH gebildet wird und $Z_2$ ein Wasserstoffatom ist, stellt man eine Verbindung der allgemeinen Formel I her, in der X und $R_1$ bis $R_7$ die in Anspruch 1 angegebenen Bedeutungen besitzen, Y ein Stickstoffatom darstellt und Z ein Schwefelatom bedeutet; oder

C) durch Reaktion einer Verbindung der allgemeinen Formel IV

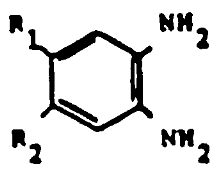

IV

mit einer Verbindung der allgemeinen Formel V

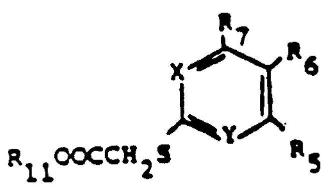

V

in denen die Radikale X, Y, $R_1$, $R_2$, $R_5$, $R_6$ und $R_7$ die in Anspruch 1 angegebenen Bedeutungen besitzen und $R_{11}$ ein Wasserstoffatom oder ein niederes Alkyl-Radikal wie Methyl oder Ethyl darstellt, stellt man eine Verbindung der allgemeinen Formel I her, in der X, Y, $R_1$, $R_2$, $R_5$, $R_6$ und $R_7$ die in Anspruch 1 angegebenen Bedeutungen besitzen, $R_3$ und $R_4$ ein Wasserstoffatom darstellen und Z ein Schwefelatom bedeutet; oder

D) durch Oxidation von Verbindungen der allgemeinen Formel I, in der X, Y und $R_1$ bis $R_7$ die in Anspruch 1 angegebenen Bedeutungen besitzen und Z ein Schwefelatom darstellt, mit einer stöchiometrischen Menge eines Oxidationsmittels, stellt man Verbindungen der Formel I her, in der X, Y und $R_1$ bis $R_7$ die in Anspruch 1 angegebenen Bedeutungen besitzen und Z eine Sulfinylgruppe (S→O) darstellt; wobei das genannte Oxidationsmittel vorzugsweise ausgewählt wird unter Wasserstoffperoxid, den Persäuren, wie meta-Chlorperbenzoesäure, Salpetersäure, Natriummetaperiodat, Chromsäure, Mangandioxid, Chlor, Brom oder Sulfurylchlorid; oder

E) durch Oxidation von Verbindungen der allgemeinen Formel I, in der X, Y und $R_1$ bis $R_7$ die in Anspruch 1 angegebenen Bedeutungen besitzen und Z ein Schwefelatom darstellt, mit einer zwei- bis dreimal höheren als der stöchiometrischen Menge eines Oxidationsmittels, stellt man Verbindungen der Formel I her, in der X, Y und $R_1$ bis $R_7$ die in Anspruch 1 angegebenen Bedeutungen besitzen und Z eine Sulfonylgruppe (O←S→O) darstellt; wobei das genannte Oxidationsmittel vorzugsweise ausgewählt wird unter Wasserstoffperoxid, den Persäuren, wie meta-Chlorperbenzoesäure, Salpetersäure, Natriummetaperiodat, Chromsäure, Mangandioxid, Chlor, Brom oder Sulfurylchlorid.

**7.** Verfahren zur Herstellung der Verbindungen nach einem der Ansprüche 1 bis 5, dadurch gekennzeichnet, daß mindestens eine der nachfolgenden Operationen durchgeführt wird:

F) Durch Nitrierung der Verbindungen der allgemeinen Formel I, in der $R_2$ und $R_3$ Wasserstoffatome darstellen und X, Y, Z, $R_1$ und $R_4$ bis $R_7$ die in Anspruch 1 angegebenen Bedeutungen besitzen, in einer Mischung von Schwefelsäure und Salpetersäure stellt man Verbindungen der allgemeinen Formel I her, in der X, Y, Z, $R_1$ und $R_4$ bis $R_7$ die in Anspruch 1 angegebenen Bedeutungen

besitzen, $R_2$ eine Nitrogruppe ($NO_2$) bedeutet und $R_3$ ein Wasserstoffatom ist; oder

G) zur Herstellung von Verbindungen der allgemeinen Formel I, in der $R_1$ oder $R_2$ ein Methoxycarbonyl-Radikal ($COOCH_3$) darstellen, $R_3$ ein Wasserstoffatom ist und $R_2$ oder $R_1$, X, Y, Z und $R_4$ bis $R_7$ die in Anspruch 1 angegebenen Bedeutungen besitzen, bringt man eine Verbindung der allgemeinen Formel I, in der $R_1$ oder $R_2$ ein Carbonsäure-Radikal (COOH) darstellen, $R_3$ ein Wasserstoffatom ist und $R_2$ oder $R_1$, X, Y, Z und $R_4$ bis $R_7$ die in Anspruch 1 angegebenen Bedeutungen besitzen, mit Methanol zur Reaktion; oder

H) zur Herstellung von Verbindungen der allgemeinen Formel I, in der $R_3$ ein niederes Alkyl-Radikal mit 1 bis 4 Kohlenstoffatomen ist und X, Y, Z, $R_1$, $R_2$ und $R_4$ bis $R_7$ die in Anspruch 1 angegebenen Bedeutungen besitzen, bringt man eine Verbindung der allgemeinen Formel I, in der $R_3$ ein Wasserstoffatom ist und X, Y, Z, $R_1$, $R_2$ und $R_4$ bis $R_7$ die in Anspruch 1 angegebenen Bedeutungen besitzen, mit einem Alkylierungsmittel zur Reaktion, wie Dimethylsulfat, Dimethylformamid-dimethylacetal oder einem Alkylhalogenid, worin die bevorzugten Halogene Chlor, Brom und Iod sind; oder

I) zur Herstellung von Verbindungen der allgemeinen Formel I, in der $R_3$ ein an ein anderes Radikal $R_9$ ($-CO-R_9$) gebundenes Carbonyl-Radikal bedeutet und X, Y, Z, $R_1$, $R_2$ und $R_4$ bis $R_9$ die in Anspruch 1 angegebenen Bedeutungen besitzen, bringt man eine Verbindung der allgemeinen Formel I, in der $R_3$ ein Wasserstoffatom ist und X, Y, Z, $R_1$, $R_2$ und $R_4$ bis $R_7$ die in Anspruch 1 angegebenen Bedeutungen besitzen, mit einer Verbindung der allgemeinen Formel VI

$Cl - CO - R_9$   VI

zur Reaktion, in der $R_9$ die in Anspruch 1 angegebene Bedeutung besitzt.

**8.** Als Medikamente die Derivate der allgemeinen Formel I und ihre therapeutisch akzeptablen Salze, nach einem der Ansprüche 1 bis 5, insbesondere als Medikamente zur Behandlung von gastro-intestinalen Erkrankungen, hauptsächlich bezeichnet als Inhibitoren der Magensäure-Sekretion und als Cytoprotektor-Mittel.

**9.** Pharmazeutische Zusammensetzungen, dadurch gekennzeichnet, daß sie außer einem pharmazeutisch akzeptablen Trägerstoff mindestens ein Derivat der allgemeinen Formel I oder eines seiner physiologisch akzeptablen Salze nach einem der Ansprüche 1 bis 5 enthalten.

**10.** Verwendung der Derivate der allgemeinen Formel I und ihrer physiologisch akzeptablen Salze nach einem der Ansprüche 1 bis 5 für die Herstellung von Medikamenten zur Behandlung von gastro-intestinalen Erkrankungen, insbesondere für die Herstellung von Inhibitor-Mitteln der Magensäure-Sekretion und von Cytoprotektor-Mitteln.

**Patentansprüche für folgende Vertragsstaaten : AT, GR**

**1.** Verfahren zur Herstellung von 2-Alkylbenzimidazol-Derivaten der allgemeinen Formel I

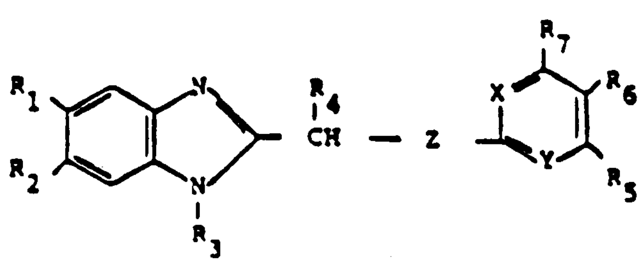

I

sowie ihrer therapeutisch akzeptablen Salze, in der

- X ein Stickstoffatom (N) oder ein an ein anderes Radikal $R_8$ ($C-R_8$) gebundenes Kohlenstoffatom darstellt;
- Y ein Stickstoffatom (N) oder eine N-Oxid-Gruppe ($N\rightarrow O$) bedeutet;
- Z ein Schwefelatom (S), eine Sulfinylgruppe ($S\rightarrow O$) oder eine Sulfonylgruppe ($O\leftarrow S\rightarrow O$) ist;

| | |
|---|---|
| $R_1$ und $R_2$, | gleich oder verschieden, ein Wasserstoffatom, ein Halogenatom, ein lineares oder verzweigtes, niederes Alkyl-Radikal mit 1 bis 4 Kohlenstoffatomen, eine Nitrogruppe ($NO_2$), eine Trifluormethylgruppe ($CF_3$), ein niederes Alkoxy-Radikal mit 1 bis 4 Kohlenstoffatomen, ein Methylcarbonyl-Radikal, ein Methoxycarbonyl-Radikal oder ein Benzoyl-Radikal darstellen; |
| $R_3$ | ein Wasserstoffatom, ein niederes Alkyl-Radikal mit 1 bis 4 Kohlenstoffatomen, ein an ein anderes Radikal $R_9$ (-CO-$R_9$) gebundenes Carbonyl-Radikal bedeutet; |
| $R_4$ | ein Wasserstoffatom oder ein niederes Alkyl-Radikal mit 1 bis 4 Kohlenstoffatomen ist; |
| $R_5$ | ein Wasserstoffatom, ein Methyl-Radikal oder ein Hydroxy-Radikal darstellt; |
| $R_6$ | ein Wasserstoffatom, ein Methyl-Radikal oder ein Nitro-Radikal ($NO_2$) bedeutet; |
| $R_7$ | ein Wasserstoffatom oder ein niederes Alkyl-Radikal mit 1 bis 4 Kohlenstoffatomen ist; |
| $R_8$ | ein Wasserstoffatom oder ein Methyl-Radikal darstellt; und |
| $R_9$ | ein Alkoxy-Radikal bedeutet, |

jedoch mit der Ausnahme der Verbindung der Formel I, in der

| | |
|---|---|
| X | CH darstellt, |
| Y | N bedeutet, |
| Z | Schwefel ist, und |
| $R_1$ bis $R_7$ | Wasserstoff darstellen, |

dadurch gekennzeichnet, daß mindestens eine der nachfolgenden Operationen durchgeführt wird:

A) Durch Reaktion einer Verbindung der allgemeinen Formel II

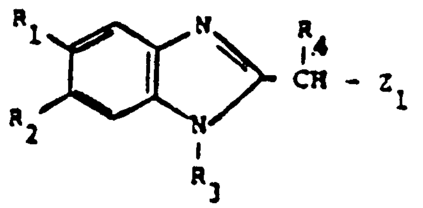

II

mit einer Verbindung der allgemeinen Formel III

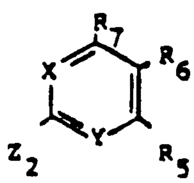

III

in denen die Radikale X, Y und $R_1$ bis $R_7$ die vorstehend angegebenen Bedeutungen besitzen und eines der zwei Radikale $Z_1$ und $Z_2$ durch das Radikal SH und das andere durch eine Abgangsgruppe gebildet wird, insbesondere ausgewählt unter den Halogenen, vorzugsweise Fluor, Chlor und Brom; durch aus veresterten Gruppen gebildeten reaktiven Radikalen, insbesondere Acetyloxy, Tosyloxy oder Mesyloxy, oder auch durch Alkylthio- oder Alkylsulfinyl-Gruppen, beispielsweise Methylthio oder Methylsulfinyl; oder

B) durch Reaktion einer Verbindung der allgemeinen Formel II mit einer Verbindung der allgemeinen Formel III, in denen X und $R_1$ bis $R_7$ die vorstehend angegebenen Bedeutungen besitzen, Y eine N-Oxid-Gruppe ($N \rightarrow O$) darstellt, $Z_1$ durch das Radikal -SH gebildet wird und $Z_2$ ein Wasserstoffatom ist, stellt man eine Verbindung der allgemeinen Formel I her, in der X und $R_1$ bis $R_7$ die vorstehend angegebenen Bedeutungen besitzen, Y ein Stickstoffatom darstellt und Z ein Schwefelatom bedeutet; oder

C) durch Reaktion einer Verbindung der allgemeinen Formel IV

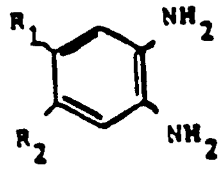

IV

mit einer Verbindung der allgemeinen Formel V

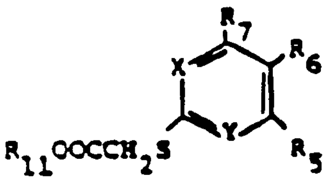

V

in denen die Radikale X, Y, $R_1$, $R_2$, $R_5$, $R_6$, und $R_7$ die vorstehend angegebenen Bedeutungen besitzen und $R_{11}$ ein Wasserstoffatom oder ein niederes Alkyl-Radikal wie Methyl oder Ethyl darstellt, stellt man eine Verbindung der allgemeinen Formel I her, in der X, Y, $R_1$, $R_2$, $R_5$, $R_6$ und $R_7$ die vorstehend angegebenen Bedeutungen besitzen, $R_3$ und $R_4$ ein Wasserstoffatom darstellen und Z ein Schwefelatom bedeutet; oder

D) durch Oxidation von Verbindungen der allgemeinen Formel I, in der X, Y und $R_1$ bis $R_7$ die vorstehend angegebenen Bedeutungen besitzen und Z ein Schwefelatom darstellt, mit einer stöchiometrischen Menge eines Oxidationsmittels, stellt man Verbindungen der Formel I her, in der X, Y und $R_1$ bis $R_7$ die vorstehend angegebenen Bedeutungen besitzen und Z eine Sulfinylgruppe ($S\rightarrow O$) darstellt; wobei das genannte Oxidationsmittel vorzugsweise ausgewählt wird unter Wasserstoffperoxid, den Persäuren, wie meta-Chlorperbenzoesäure, Salpetersäure, Natriummetaperiodat, Chromsäure, Mangandioxid, Chlor, Brom oder Sulfurylchlorid; oder

E) durch Oxidation von Verbindungen der allgemeinen Formel I, in der X, Y und $R_1$ bis $R_7$ die vorstehend angegebenen Bedeutungen besitzen und Z ein Schwefelatom darstellt, mit einer zwei- bis dreimal höheren als der stöchiometrischen Menge eines Oxidationsmittels, stellt man Verbindungen der Formel I her, in der X, Y und $R_1$ bis $R_7$ die vorstehend angegebenen Bedeutungen besitzen und Z eine Sulfonylgruppe ($O\leftarrow S\rightarrow O$) darstellt; wobei das genannte Oxidationsmittel vorzugsweise ausgewählt wird unter Wasserstoffperoxid, den Persäuren, wie meta-Chlorperbenzoesäure, Salpetersäure, Natriummetaperiodat, Chromsäure, Mangandioxid, Chlor, Brom oder Sulfurylchlorid;

wobei die durch die vorstehenden Verfahren erhaltenen Produkte der allgemeinen Formel I gegebenenfalls später umgewandelt werden können.

**2.** Verfahren nach Anspruch 1, dadurch gekennzeichnet, daß die späteren Umwandlungen mindestens unter Anwendung einer der folgenden Operationen durchgeführt werden:

F) Durch Nitrierung der Verbindungen der allgemeinen Formel I, in der $R_2$ und $R_3$ Wasserstoffatome darstellen und X, Y, Z, $R_1$ und $R_4$ bis $R_7$ die vorstehend angegebenen Bedeutungen besitzen, in einer Mischung von Schwefelsäure und Salpetersäure stellt man Verbindungen der allgemeinen Formel I her, in der X, Y, Z, $R_1$ und $R_4$ bis $R_7$ die vorstehend angegebenen Bedeutungen besitzen, $R_2$ eine Nitrogruppe ($NO_2$) bedeutet und $R_3$ ein Wasserstoffatom ist; oder

G) zur Herstellung von Verbindungen der allgemeinen Formel I, in der $R_1$ oder $R_2$ ein Methoxycarbonyl-Radikal ($COOCH_3$) darstellen, $R_3$ ein Wasserstoffatom ist und $R_2$ oder $R_1$, X, Y, Z und $R_4$ bis $R_7$ die vorstehend angegebenen Bedeutungen besitzen, bringt man eine Verbindung der allgemeinen Formel I, in der $R_1$ oder $R_2$ ein Carbonsäure-Radikal (COOH) darstellen, $R_3$ ein Wasserstoffatom ist und $R_2$ oder $R_1$, X, Y, Z und $R_4$ bis $R_7$ die vorstehend angegebenen Bedeutungen besitzen, mit Methanol zur Reaktion; oder

H) zur Herstellung von Verbindungen der allgemeinen Formel I, in der $R_3$ ein niederes Alkyl-Radikal mit 1 bis 4 Kohlenstoffatomen ist und X, Y, Z, $R_1$, $R_2$ und $R_4$ bis $R_7$ die vorstehend angegebenen Bedeutungen besitzen, bringt man eine Verbindung der allgemeinen Formel I, in der $R_3$ ein Wasserstoffatom ist und X, Y, Z, $R_1$, $R_2$ und $R_4$ bis $R_7$ die vorstehend angegebenen Bedeutungen besitzen, mit einem Alkylierungsmittel zur Reaktion, wie Dimethylsulfat, Dimethylformamid-dimethylacetal oder einem Alkylhalogenid, worin die bevorzugten Halogene Chlor, Brom und Iod sind; oder
I) zur Herstellung von Verbindungen der allgemeinen Formel I, in der $R_3$ ein an ein anderes Radikal $R_9$ (-CO-$R_9$) gebundenes Carbonyl-Radikal bedeutet und X, Y, Z, $R_1$, $R_2$ und $R_4$ bis $R_9$ die vorstehend angegebenen Bedeutungen besitzen, bringt man eine Verbindung der allgemeinen Formel I, in der $R_3$ ein Wasserstoffatom ist und X, Y, Z, $R_1$, $R_2$ und $R_4$ bis $R_7$ die vorstehend angegebenen Bedeutungen besitzen, mit einer Verbindung der allgemeinen Formel VI

Cl - CO - $R_9$ VI

zur Reaktion, in der $R_9$ die vorstehend angegebene Bedeutung besitzt.